(19) Europäisches Patentamt
European Patent Office
Office européen des brevets

(11) EP 0 567 967 B1

(12) **EUROPÄISCHE PATENTSCHRIFT**

(45) Veröffentlichungstag und Bekanntmachung des Hinweises auf die Patenterteilung:
**10.07.1996 Patentblatt 1996/28**

(21) Anmeldenummer: **93106725.0**

(22) Anmeldetag: **26.04.1993**

(51) Int Cl.6: **C07D 401/12**, A61K 31/395, C07D 207/263, C07D 207/08, C07D 233/72, C07D 233/32, C07D 249/12, C07D 285/10, C07D 403/06, C07D 211/34, C07C 257/18

(54) **Tritiumarkierte Fibrinogen-Rezeptor-Antagonisten, deren Verwendung und Verfahren zur ihrer Herstellung**

Tritium-labelled fibrinogen-receptor-antagonists, their use and their production

Composés marqués au tritium utilisé comme antagonistes de récepteur de fibrinogen et leur préparation

(84) Benannte Vertragsstaaten:
**AT BE CH DE DK ES FR GB GR IE IT LI LU NL PT SE**

(30) Priorität: **28.04.1992 DE 4213930**
**30.04.1992 DE 4214245**

(43) Veröffentlichungstag der Anmeldung:
**03.11.1993 Patentblatt 1993/44**

(73) Patentinhaber: **Dr. Karl Thomae GmbH**
**D-88397 Biberach (DE)**

(72) Erfinder:
• **Weisenberger, Johannes, Dr.**
  **W-7950 Biberach 1 (DE)**

• **Schubert, Hans-Dieter**
  **W-7950 Biberach 1 (DE)**
• **Switek, Karl-Heinz**
  **W-7950 Biberach 1 (DE)**
• **Linz, Günter, Dr.**
  **W7951 Mittelbiberach (DE)**
• **Himmelsbach, Frank, Dr.**
  **W-7951 Mittelbiberach (DE)**

(56) Entgegenhaltungen:
**EP-A- 0 381 033      EP-A- 0 483 667**
**EP-A- 0 496 378      EP-A- 0 528 369**

• **J.MED.CHEM. 4. Mai 1992, Seiten 4393-4407, ALIG et al.: "Low Molecular Weight, Non-Peptide Fibrinogen Receptor Antagonists".**

**Beschreibung**

Im Normalfall, d.h. bei intakten Blutgefäßen, ist die Aufrechterhaltung der Fließfähigkeit des Blutes dadurch gewährleistet, daß keiner der maßgeblichen hämostatischen Mechanismen aktiviert ist. Zur Blutstillung bei Verletzungen werden folgende zwei Prozesse eingeleitet:

1. Die unmittelbar an der Gefäßläsion innerhalb von Sekunden einsetzende Thrombozytenaggregation, welche durch den sich bildenden Thrombus die Blutstillung initiiert, und

2. die etwas später eintretende Gerinnung, welche den Thrombus durch Bildung von Fibrinfäden stabilisiert.

Die Aggregation und die Gerinnung können nun auch bei nicht verletzten, aber etwa durch atherosklerotische Plaques veränderten Gefäßwänden auftreten und verursachen so lebensbedrohende Krankheiten wie Herzinfarkt, Lungenembolie und Gehirnschlag.

Eine logisch erscheinende Präventivmaßnahme wäre in diesen Fällen die Verhinderung der initialen Thrombusbildung. Verschiedene Pharmazeutika, welche einzelne Wege der Thrombozytenaggregation hemmen, wurden bisher ohne durchschlagenden Erfolg hierfür eingesetzt. Die gemeinsame Endstufe aller Aktivierungswege ist die Bildung des Thrombus, welcher nach neuesten Erkenntnissen dadurch entsteht, daß Thrombozyten über die an ihrer Membran vorhandenen Fibrinogen-Rezeptoren durch das bifunktionale, fadenförmige Fibrinogen zusammengehalten werden. Wenn es gelänge, diesen Vorgang zu unterbinden, könnte die Thrombusbildung - unabhängig von der Art der Aktivierung - gehemmt werden (siehe Cahill, M. et al. in Brit. J. Clin. Pharmcol. 33, 3-9 (1992)).

Zur Suche nach Fibrinogen-Rezeptor-Antagonisten, welche den Fibrinogen-Rezeptor blockieren und somit die Bindung des Fibrinogens an die Thrombozyten hemmen, wird eine Methode benötigt, um diese Bindung quantitativ bestimmen zu können. Eine Standardmethode ist die Verwendung von $^{125}$J-Fibrinogen und die Trennung von zellgebundenem und gelöstem Fibrinogen durch Zentrifugation. Dieses Verfahren hat folgende gravierende Nachteile:

1. Das verwendete Nukleotid ist ein γ-Strahler
2. Die Halbwertszeit beträgt nur 60 Tage
3. Fibrinogen ist ein adhäsives Protein
4. Die Bindung ist nicht vollständig reversibel
5. Die Methode ist nicht anwendbar in Gegenwart von Plasma
6. Die Thrombozyten müssen aktiviert werden

Für den praktischen Einsatz sind die Punkte 4 und 6 nicht sehr wichtig. Die Punkte 1 bis 3 bedingen jedoch umfangreiche Abschirmmaßnahmen, erschweren die Vorratshaltung bzw. bedingen Materialverluste und lassen die Verwendung von Pipettierautomaten als kritisch erscheinen. Der größte Nachteil ist jedoch, daß die Methode in Gegenwart von Plasma infolge des hohen Fibrinogengehalts nicht anwendbar ist, da Plasma-Fibrinogen die Bindung von $^{125}$J-Fibrinogen an aktivierte Plättchen mit einer $IC_{50}$ von 120 nM hemmt. Zur Messung der Bindung müssen daher gewaschene bzw. gelfiltrierte Thrombozyten verwendet werden (siehe Harfenist, E.J. et al. in Blood 71, 132-136 (1988)).

Überraschenderweise wurde nun gefunden, daß es Fibrinogen-Rezeptor-Antagonisten gibt, die auch in Gegenwart von Plasma-Fibrinogen an den Fibrinogen-Rezeptor binden. Derartige Fibrinogen-Rezeptor-Antagonisten werden beispielsweise in der EP-A-0,372,486 und in der EP-A-0,381,033 sowie in den nicht vorveröffentlichten deutschen bzw. europäischen Offenlegungsschriften EP-A-0 483 667 (Case 5/1054), EP-A-0 496 378 (Case 5/1056), EP-A-0 503 548 (Case 5/1068), EP-A-0 525 629 (Case 5/1074), EP-A-O 528 369 (Case 5/1075), DE-A-41 29 603 (Case 5/1076) und DE-A-41 34 467 (Case 5/1077) beschrieben.

Bevorzugte Fibrinogen-Rezeptor-Antagonisten sind die Amidine der allgemeinen Formel

$$R_a - R_b, \hspace{4cm} (I)$$

in der

$R_a$ eine 4-Amidinophenyl- oder 5-Amidino-pyrimid-2-yl-Gruppe und
$R_b$ eine HOOC-D-C-B-A-Gruppe bedeuten, in der

A eine gegebenenfalls durch eine Methoxygruppe substituierte Phenylengruppe, in der zusätzlich eine oder zwei Methingruppen jeweils durch ein Stickstoffatom ersetzt sein können, eine gegebenenfalls an einem Kohlenstoffatom durch eine Methyl-, Ethyl- oder Trifluormethylgruppe substituierte Imidazolinon-di-yl-, Imidazolidinon-di-yl-, Imidazolidin-dion-di-yl-, Triazolinon-di-yl- oder 1,1-Dioxo-1,2,5-thiadiazolidin-di-yl-gruppe, eine gegebenenfalls an einem der Stickstoffatome durch den Rest $R_1$ substituierte Benzimidazol-di-yl-gruppe oder eine mit dem Stickstoffatom an den Rest B gebundene Aminocarbonylgruppe,

B eine Methylen-, Carbonyl-, Cyclohexylen-, Phenylen- oder Imidazol-di-yl-gruppe, eine über das Stickstoffatom an den Rest C gebundene Aminocarbonylgruppe, welche gleichzeitig am Stickstoffatom durch eine Methylgruppe substituiert sein kann, oder eine über das Sauerstoffatom an den Rest A gebundene Methylenoxygruppe,

C eine gegebenenfalls durch den Rest $R_2$ substituierte Ethylengruppe, eine Cyclohexylengruppe, eine gegebenenfalls am Stickstoffatom durch den Rest $R_3$ substituierte Pyrrolidin-di-yl- oder Pyrrolidinon-di-yl-gruppe, eine Piperidin-di-yl-gruppe oder eine mit dem Stickstoffatom an den Rest D gebundene Aminocarbonylgruppe und

D eine Bindung, eine Methylen- oder Ethylengruppe darstellen, wobei

$R_1$ eine Methyl-, 2-Piperazinoethyl-, 2-(3,4-Dimethoxyphenyl)ethyl- oder 3-Thiomorpholinopropylgruppe,
$R_2$ eine Amino- oder Hydroxygruppe und
$R_3$ eine 3-Phenylpropyl-, Acetyl-, Methansulfonyl- oder Pyrrolidinocarbonylmethylgruppe darstellen,

insbesondere diejenigen Fibrinogen-Rezeptor-Antagonisten, in denen

$R_a$ eine 4-Amidino-phenylgruppe oder auch, wenn $R_b$ eine in 4-Stellung durch eine 3-Carboxymethyl-pyrrolidin-2-on-5-yl-methyloxygruppe substituierte Phenylgruppe darstellt, eine 5-Amidino-pyrimid-2-yl-Gruppe und
$R_b$ eine Phenylgruppe, die in 4-Stellung durch eine 4-Carboxymethyl-pyrrolidin-2-yl-methyloxy-, 3-Carboxymethyl-pyrrolidin-2-on-5-yl-methyloxy-, 4-Carboxymethyl-piperidinomethyl-, 4-Carboxymethyl-piperidinocarbonyl-, 4-Carboxycyclohexylaminocarbonyl- oder N-Methyl-N-(4-carboxycyclohexyl)aminocarbonylgruppe substituiert ist, wobei jeweils in 1-Stellung der Pyrrolidinteil durch eine Acetyl- oder Methansulfonylgruppe und der Pyrrolidinonteil durch eine 3-Phenylpropyl- oder Pyrrolidinocarbonylmethylgruppe substituiert sein kann,

eine 4-Methoxy-phenylgruppe, die in 3-Stellung durch eine 4-Carboxycyclohexyl-aminocarbonylgruppe substituiert ist,

eine in 6-Stellung durch eine Imidazol-I-yl-Gruppe substituierte Pyridazin-3-yl-Gruppe, wobei der Imidazolylteil in 4-Stellung durch eine 2-Amino-2-carboxy-ethyl- oder 2-Carboxy-2-hydroxy-ethylgruppe substituiert ist,

eine Imidazolidin-2-on-1-yl-Gruppe, die in 3-Stellung durch eine 4-(2-Carboxyethyl)-cyclohexyl- oder 4-(2-Carboxyethyl)phenylgruppe substituiert ist,

eine 4-Methyl-4-imidazolin-2-on-1-yl- oder Imidazolidin-2,4-dion-1-yl-Gruppe, die in 3-Stellung durch eine 4-(2-Carboxyethyl)-phenyl-Gruppe substituiert ist,

eine gegebenenfalls in 5-Stellung durch eine Methyl- oder Ethylgruppe substituierte 1,2,4-Triazol-5-in-3-on-2-yl-Gruppe, die in 4-Stellung durch eine 4-(2-Carboxyethyl)-phenylgruppe substituiert ist,

eine 1,2,4-Triazol-5-in-3-on-4-yl-Gruppe, die in 2-Stellung durch eine 4-(2-Carboxyethyl)-phenyl-Gruppe substituiert ist,

eine in 5-Stellung durch eine 4-(2-Carboxyethyl)-phenylgruppe substituierte 1,1-Dioxo-1,2,5-thiadiazolidin-2-yl-Gruppe,

eine gegebenenfalls in 1-Stellung durch eine Methyl-, 2-Piperazino-ethyl- oder 2-(3,4-Dimethoxyphenyl)-ethylgruppe substituierte Benzimidazol-2-yl-Gruppe, die in 5-Stellung durch eine 2-Carboxyethylaminocarbonylgruppe substituiert ist, oder

eine Phenylaminocarbonylgruppe, die in 3-Stellung durch eine 2-Carboxyethylaminocarbonylgruppe substituiert ist, bedeuten,

deren Tautomere, deren Stereoisomere einschließlich deren Gemische und deren Salze.

Besonders bevorzugt sind jedoch diejenigen Verbindungen der obigen allgemeinen Formel I, welche eine gute in vitro-Wirkung im Collagen-induzierten Aggregationstest aufweisen, wobei die erfindungsgemäßen mit Tritium markierten Fibrinogen-Rezeptor-Antagonisten in diesem Test (siehe Huang, E.M. und Detwiler, T.C.: "Stimulus-Response Coupling Mechanisms" in Biochemistry of Platelets, Academic Press, Orlando, Florida 1986, Seiten 1-68) eine $EC_{50}$-Wirkung von weniger als 500 nM aufweisen, insbesondere jedoch die Verbindungen

(1) (3S,5S)- und (3R,5R)-5-[[(4-(5-Amidinopyrimid-2-yl)-phenyl]-oxymethyl]-3-carboxymethyl-pyrrolidin-2-on,

(2) (3S,5S)- und (3R,5R)-1-Acetyl-5-[(4'-amidino-4-biphenylyl)-oxymethyl]-3-carboxymethyl-pyrrolidin,

(3) (3S,5S)- und (3R,5R)-5-[(4'-Amidino-4-biphenylyl)-oxymethyl]-3-carboxymethyl-1-methansulfonyl-pyrrolidin,

(4) (3S,5S)- und (3R,5R)-5-[(4'-Amidino-4-biphenylyl)-oxymethyl]-3-carboxymethyl-pyrrolidin-2-on,

(5) 4-Amidino-4'-[(trans-4-carboxycyclohexyl)-aminocarbonyl]biphenyl,

(6) 4-Amidino-4'-[N-(trans-4-carboxycyclohexyl)-N-methylaminocarbonyl]-biphenyl,

(7) 1-(4-Amidinophenyl)-3-[4-(2-carboxyethyl)-phenyl]-imidazolidin-2,4-dion,

(8) 1-(4-Amidinophenyl)-3-[4-(2-carboxyethyl)-phenyl]-4-methyl-4-imidazolin-2-on,

(9) 2-(4-Amidinophenyl)-4-[4-(2-carboxyethyl)-phenyl]-1,2,4-triazol-5-in-3-on,

(10) 4-(4-Amidinophenyl)-2-[4-(2-carboxyethyl)-phenyl]-1,2,4-triazol-5-in-3-on,

(11) 2-(4-Amidinophenyl)-4-[4-(2-carboxyethyl)-phenyl]-5-methyl-1,2,4-triazol-5-in-3-on,

(12) 2-(4-Amidinophenyl)-4-[4-(2-carboxyethyl)-phenyl]-5-ethyl-1,2,4-triazol-5-in-3-on,

(13) 2-(4-Amidinophenyl)-5-[4-(2-carboxyethyl)-phenyl]-1,2,5-thiadiazolidin-1,1-dioxid,

(14) 1-(4-Amidinophenyl)-3-[4-(2-carboxyethyl)-cyclohexyl]imidazolidin-2-on,

(15) 2-(4-Amidinophenyl)-5-[(2-carboxyethyl)-aminocarbonyl]-1-[2-(piperazin-1-yl)-ethyl]-benzimidazol,

(16) 4-Amidino-4'-[4-(carboxymethyl)-piperidinocarbonyl]-biphenyl und

deren Salze.
Ganz besonders bevorzugte Verbindungen sind

(1) (3S,5S)- und (3R,5R)-5-[[(4-(5-Amidinopyrimid-2-yl)-phenyl]-oxymethyl]-3-carboxymethyl-pyrrolidin-2-on,

(2) (3S,5S)- und (3R,5R)-1-Acetyl-5-[(4'-amidino-4-biphenyl-yl)-oxymethyl]-3-carboxymethyl-pyrrolidin,

(3) (3S,5S)- und (3R,5R)-5-[(4'-Amidino-4-biphenylyl)-oxymethyl]-3-carboxymethyl-l-methansulfonyl-pyrrolidin,

(4) (3S,5S)- und (3R,5R)-5-[(4'-Amidino-4-biphenylyl)-oxymethyl]-3-carboxymethyl-pyrrolidin-2-on,

(5) 4-Amidino-4'-[4-(carboxymethyl)-piperidinocarbonyl]-bi-phenyl und

deren Salze.
Die vorstehend erwähnten Fibrinogen-Rezeptor-Antagonisten der allgemeinen Formel I weisen eine vergleichbare oder höhere Affinität gegenüber dem Rezeptor als $^{125}$J-Fibrinogen auf. Ihre Bindung an den Rezeptor wird durch Fremdproteine nicht gestört. Sie können daher, wenn in ihnen mindestens ein Wasserstoffatom, vorzugsweise ein aromatisches Wasserstoffatom, durch ein Tritiumatom ersetzt ist, als Liganden im Fibrinogen-Rezeptor-Bindungstest auch in Gegenwart von Plasma eingesetzt werden.
Gegenstand der vorliegenden Erfindung ist somit die Verwendung von Tritium markierten Fibrinogen-Rezeptor-Antagonisten, die eine vergleichbare oder höhere Affinität gegenüber dem Rezeptor wie $^{125}$J-Fibrinogen besitzen und deren Bindung durch Fremdproteine nicht gestört wird sowie in Gegenwart von Fremdprotein, z.B. von Albumin oder von Fibrinogen, eine Affinität ($K_D$) von weniger als 500 nM gegenüber dem Rezeptor aufweisen, als Liganden

a) zur Bestimmung der Bindung von chemischen Substanzen an Fibrinogen-Rezeptoren und

b) zur Konzentrationsbestimmung von Fibrinogen-Rezeptor-Antagonisten

jeweils insbesondere in Gegenwart von Fremdeiweiß und/oder in unterschiedlichen Körperflüssigkeiten wie Blutplasma oder Urin und Verfahren zu ihrer Herstellung.
Ein weiterer Gegenstand sind die neuen Tritium markierten Fibrinogen-Rezeptor-Antagonisten der vorstehend erwähnten allgemeinen Formel I.

Der erfindungsgemäße Fibrinogen-Rezeptor-Bindungstest wurde beispielsweise unter Verwendung von (3S,5S)-5-[(4'-Amidino-4-biphenylyl)-oxymethyl]-3-carboxymethyl-pyrrolidin-2-on-[3-$^3$H-4-biphenylyl] [= $^3$H-BIBU 52 ZW] als Ligand wie folgt durchgeführt:

Eine Suspension von Humanthrombozyten in Plasma wird mit $^3$H-BIBU 52 und verschiedenen Konzentrationen der zu testenden Substanz inkubiert. Der freie und gebundene Ligand wird durch Zentrifugation getrennt und durch Szintillationszählung quantitativ bestimmt. Aus den Meßwerten wird die Hemmung der $^3$H-BIBU 52-Bindung durch die Testsubstanz bestimmt.

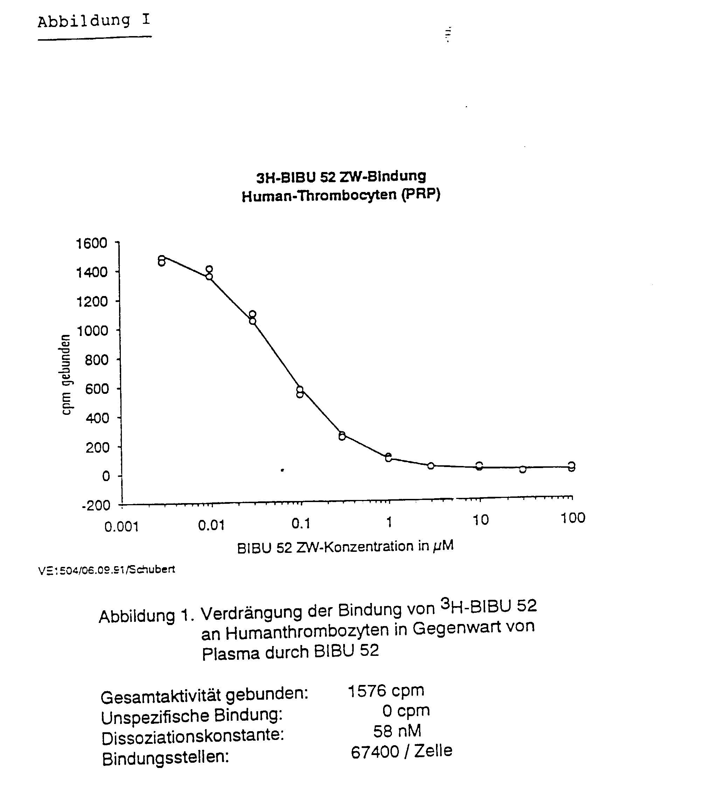

Um beispielsweise die Kompetition der Bindung von $^3$H-BIBU 52 durch nichtmarkiertes BIBU 52 zu messen, wird aus einer Antikubitalvene Spenderblut entnommen und mit Trinatriumzitrat antikoaguliert (Endkonzentration 13 mM). Das Blut wird 10 Minuten bei 170 x g zentrifugiert und das überstehende plättchenreiche Plasma (PRP) abgenommen. Das Restblut wird zur Gewinnung von Plasma noch einmal scharf abzentrifugiert. Das PRP wird mit autologem Plasma 1:10 verdünnt. 750 µl werden mit 50 µl physiologischer Kochsalzlösung, 100 µl Testsubstanzlösung, 50 µl $^{14}$C-Sucrose und 50 µl $^3$H-BIBU 52 bei Raumtemperatur 20 Minuten inkubiert. Zur Messung der unspezifischen Bindung wird anstelle der Testsubstanz 30 µM BIBU 52 eingesetzt. Die Proben werden 20 Sekunden bei 10000 x g zentrifugiert und der Überstand abgezogen. 100 µl hiervon werden zur Bestimmung des freien Liganden gemessen. Das Pellet wird in 500 µl 0.2N NaOH gelöst, 450 µl werden mit 25 µl 5N HCl versetzt und gemessen. Das im Pellet noch verbliebene Restplasma wird aus dem $^{14}$C-Gehalt bestimmt, der gebundene Ligand aus der $^3$H-Messung. Nach Abzug der unspezifischen Bindung wird die Pelletaktivität gegen die Konzentration der Testsubstanz aufgetragen (siehe Abbildung 1) und die Konzentration für eine 50%ige Hemmung der Bindung ermittelt.

Die neuen mit Tritium markierten Fibrinogen-Rezeptor-Antagonisten erhält man nach literaturbekannten Verfahren, z. B.

a) durch katalytische Tritiierung einer ungesättigten Kohlenstoff-Kohlenstoff-Bindung mit Tritium,

b) durch hydrogenolytischen Austausch eines Halogenatoms, z. B. eines Chlor-, Brom- oder Jodatoms, gegen Tritium,

c) durch katalytischen Wasserstoff/Tritiumaustausch mit Tritium,

d) durch katalytischen Wasserstoff/Tritiumaustausch in tritiierten Lösungsmitteln oder

e) durch Tritiierung einer Vorstufe des herzustellenden Fibrinogen-Rezeptor-Antagonisten und anschließende Synthese des Fibrinogen-Rezeptor-Antagonisten.

Ein gegebenenfalls so erhaltenes Isomerengemisch kann anschließend gewünschtenfalls in seine Enantiomeren aufgetrennt und/oder
eine so erhaltene tritiierte Verbindung in ihr Salz übergeführt werden.

Das Verfahren a) wird zweckmäßigerweise in einem Lösungsmittel wie Methanol oder Ethanol, vorzugsweise jedoch in einem unpolaren Lösungsmittel wie Cyclohexan, Dioxan, Tetrahydrofuran, Ethylacetat oder Dimethylsulfoxid in Gegenwart eines geeigneten Katalysators wie Palladium/Aktivkohle, Palladiummohr, Platin/Aktivkohle, Raney-Nickel oder Gemischen aus Platin und Rhodium, Ruthenium, Osmium oder Iridium auf Aktivkohle mit Tritiumgas zweckmäßigerweise bei Raumtemperatur und unter Normaldruck bis zum Stillstand der Gasaufnahme durchgeführt. Anschließend wird zur Entfernung des labilen Tritiums vom Katalysator abfiltriert, das Lösungsmittel abgezogen, der Rückstand in einem polaren Lösungsmittel aufgenommen, 10 Minuten zum Rückfluß erhitzt und nach 12 Stunden bei Raumtemperatur das Lösungsmittel erneut abgezogen.

Das Verfahren b) wird vorzugsweise in einem geeigneten Lösungsmittel wie Wasser, Methanol, Ethanol, Dimethylformamid oder Dimethylsulfoxid, vorzugsweise jedoch in einem unpolaren Lösungsmittel wie Ethylacetat, Dioxan oder Tetrahydrofuran, in Gegenwart eines geeigneten Katalysators wie 5 % Palladium auf Aktivkohle, 10 % Palladium auf Aktivkohle, Palladium, Platin, Palladiumdichlorid, 2 % Palladium auf Bariumcarbonat, Palladium/Bariumsulfat oder Raney-Nickel unter Zusatz eines Überschusses einer basischen Komponente wie Triethylamin, Pyridin, Chinolin, Natriumhydroxid, Kaliumcarbonat, Ammoniak, Magnesiumoxid oder Kalziumoxid mit Tritiumgas bei 1 bis 5 bar und bei 20 bis 50°C, vorzugsweise bei 25°C, durchgeführt. Anschließend wird zur Entfernung des labilen Tritiums vom Katalysator abfiltriert, das Lösungsmittel abgezogen, der Rückstand in einem polaren Lösungsmittel aufgenommen, 10 Minuten zum Rückfluß erhitzt und nach 12 Stunden bei Raumtemperatur des Lösungsmittel erneut abgezogen.

Das Verfahren c) wird vorzugsweise in einem geeigneten Lösungsmittel wie Phosphat-Puffer pH 7, Wasser, Eisessig, Methanol oder Ethanol in Gegenwart eines geeigneten Katalysators wie Palladiumdioxid/Bariumsulfat, Platin, Palladium, 5 % Palladium auf Aktivkohle, 10 % Palladium auf Aktivkohle, Platindioxidhydrat oder Palladium/Platindioxidhydrat mittels Rühren unter trägerfreiem Tritiumgas innerhalb von einer Stunde bis 20 Tage bei 20 bis 50°C, vor-

EP 0 567 967 B1

zugsweise bei 25°C, durchgeführt. Anschließend wird zur Entfernung des labilen Tritiums vom Katalysator abfiltriert, das Lösungsmittel abgezogen, der Rückstand in einem polaren Lösungsmittel aufgenommen, 10 Minuten zum Rückfluß erhitzt und nach 12 Stunden bei Raumtemperatur des Lösungsmittel erneut abgezogen und das so erhaltene Produkt mit Hilfe der HPLC gereinigt.

Das Verfahren d) wird in einem geeigneten tritiierten Lösungsmittel wie HTO, 70%igem $CH_3COOT$, $CF_3COOT$, $KOH/HTO$, Dioxan/HTO, $HTSO_4/HTO/Wasser$, $CH_3OT$ oder $CH_3CH_2OT$ in Gegenwart eines geeigneten Katalysators wie Platindioxid, 5 % Palladium auf Aktivkohle, 10 % Palladium auf Aktivkohle, Palladium, Platin oder Raney-Nickel durch Erwärmen in einem geschlossenen Gefäß für ein bis 20 Stunden auf 100 bis 140°C durchgeführt. Anschließend wird zur Entfernung des labilen Tritiums vom Katalysator abfiltriert, das Lösungsmittel abgezogen, der Rückstand in einem polaren Lösungsmittel aufgenommen, 10 Minuten zum Rückfluß erhitzt und nach 12 Stunden bei Raumtemperatur des Lösungsmittel wieder abgezogen und das so erhaltene Produkt mit Hilfe der HPLC gereinigt.

Die Vorstufen zur Herstellung tritiierter Fibrinogen-Rezeptor-Antagonisten nach dem Verfahren e) werden nach üblichen chemischen Methoden hergestellt, wobei jeweils die erforderliche markierte Zwischenstufe nach den Verfahren a) bis d), vorzugsweise jedoch nach Verfahren a), hergestellt wird.

Bei den vorstehend beschriebenen Umsetzungen ist es besonders vorteilhaft anstelle der freien Carbonsäure jeweils deren Ester mit einem niederen Alkohol, z.B. deren Methyl-, Ethyl-, n-Propyl-, Isopropyl- oder tert.Butylester, insbesondere den Methylester einzusetzen. Nach der Tritiierung wird dann der Ester mittels Hydrolyse, vorzugsweise in Gegenwart einer Base wie Natronlauge, z. B. in Gegenwart von Methanol/ 2N Natronlauge (6:1) bei Raumtemperatur, in die entsprechende freie Carbonsäure übergeführt.

Besonders vorteilhaft erhält man die Verbindungen

[3H] 1-(4-Amidinophenyl)-3-[4-(2-carboxyethyl)-cyclohexyl]imidazolidin-2-on, ausgehend von 1-(4-Amidinophenyl)-3-[4-(2-carboxyethyl)-cyclohexyl]-4-imidazolin-2-on,

[3H] 1-(4-Amidinophenyl)-3-[4-(2-carboxyethyl)-phenyl]-imidazolidin-2,4-dion, ausgehend von 1-(4-Amidinophenyl)-3-[4-(2-carboxyethenyl)-phenyl]-imidazolidin-2,4-dion und

[3H] 2-(4-Amidinophenyl)-5-[4-(2-carboxyethyl)-phenyl]1,2,5-thiadiazolidin-1,1-dioxid, ausgehend von 2-(4-Amidinophenyl)-5-[4-(2-carboxyethenyl)-phenyl]-1,2,5-thiadiazol-1,1-dioxid,

durch katalytische Hydrierung gemäß Verfahren a) in Methanol oder Dimethylsulfoxid mit Tritiumgas unter Verwendung von 10 % Palladium auf Aktivkohle als Katalysator, die Verbindungen

[3H] (3S,5S)- und (3R,5R)-5-[[(4-(5-Amidinopyrimid-2-yl)phenyl]-oxymethyl]-3-carboxymethyl-pyrrolidin-2-on,

[3H] (3S,5S)- und (3R,5R)-1-Acetyl-5-[(4'-amidino-4-biphenylyl)-oxymethyl]-3-carboxymethyl-pyrrolidin,

[3H] (3S,5S)- und (3R,5R)-5-[(4'-Amidino-4-biphenylyl)oxymethyl]-3-carboxymethyl-1-methansulfonyl-pyrrolidin,

[3H] (3S,5S)- und (3R,5R)-5-[(4'-Amidino-4-biphenylyl)oxymethyl]-3-carboxymethyl-pyrrolidin-2-on,

[3H] 4-Amidino-4'-[(trans-4-carboxycyclohexyl)-aminocarbonyl]-biphenyl,

[3H] 4-Amidino-4'-[N-(trans-4-carboxycyclohexyl)-N-methylaminocarbonyl]-biphenyl,

[3H] 4-Amidino-4'-[4-(carboxymethyl)-piperidinocarbonyl]biphenyl und

[3H] 2-(4-Amidinophenyl)-5-[(2-carboxyethyl)-aminocarbonyl]-1-[2-(piperazin-1-yl)-ethyl]benzimidazol,

durch hydrogenolytischen Austausch von Halogen gegen Tritium gemäß Verfahren b) in einem geschlossenen Gefäß in Dimethylformamid unter kräftigem Rühren bei einem Umgebungsdruck von 370 GBq trägerfreiem Tritiumgas, die Verbindungen

[3H] 4-Amidino-4'-[(trans-4-carboxycyclohexyl)-aminocarbonyl]-biphenyl,

[3H] 4-Amidino-4'-[4-(carboxymethyl)-piperidinocarbonyl]biphenyl und

[3H] 4-Amidino-4'-[N-(trans-4-carboxycyclohexyl)-N-methylaminocarbonyl]-biphenyl

6

durch katalytischen Wasserstoff/Tritiumaustausch in einem tritiierten Lösungsmittel gemäß Verfahren d) durch 20-stündige Behandlung der untriierten Verbindung in Dioxan/HTO (1:1) in Gegenwart von Platindioxid bei 100°C, die Verbindungen

[3H] 1-(4-Amidinophenyl)-3-[4-(2-carboxyethyl)-phenyl]4-methyl-4-imidazolin-2-on,

[3H] 2-(4-Amidinophenyl)-4-[4-(2-carboxyethyl)-phenyl]1,2,4-triazol-5-in-3-on,

[3H] 4-(4-Amidinophenyl)-2-[4-(2-carboxyethyl)-phenyl]-1,2,4-triazol-5-in-3-on,

[3H] 2-(4-Amidinophenyl)-4-[4-(2-carboxyethyl)-phenyl]-5-methyl-1,2,4-triazol-5-in-3-on und

[3H] 2-(4-Amidinophenyl)-4-[4-(2-carboxyethyl)-phenyl]-5-ethyl-1,2,4-triazol-5-in-3-on,

durch Tritiierung einer Vorstufe des herzustellenden Fibrinogen-Rezeptor-Antagonisten und anschließende Synthese des Fibrinogen-Rezeptor-Antagonisten gemäß Verfahren e), vorzugsweise durch Behandlung einer Lösung von einem entsprechend substituierten 4-Amino-zimtsäure-methylester in Ethylacetat mit 370 GBq trägerfreiem Tritiumgas in Anwesenheit von 10 % Palladium auf Aktivkohle und anschließende Umsetzung nach bekannten Methoden zu den 1,2,4-Triazol-5-in-3-onen bzw. 4-Imidazolin-2-onen,

wobei die so bevorzugt erhaltenen Methylester anschließend bei Raumtemperatur mit Methanol/2N Natronlauge hydrolysiert werden.

Die Verbindungen (3S,5S)-5-[(4'-Amidino-4-biphenylyl)-oxymethyl]-3-carboxymethyl-pyrrolidin-2-on[3-3H-4-biphenylyl] und

(3R,5R)-5-[(4'-Amidino-4-biphenylyl)-oxymethyl]-3-carboxymethyl-pyrrolidin-2-on[3-3H-4-biphenylyl] erhält man vorzugsweise gemäß Verfahren b) durch hydrogenolytischen Austausch eines Halogenatoms in 3-Stellung des Biphenylkerns, zweckmäßigerweise des Bromatoms.

Die nachfolgenden Beispiele sollen die Erfindung näher erläutern:

Herstellung der Ausgangsverbindungen:

Beispiel A

3-Brom-4'-cyano-4-hydroxybiphenyl

3,9 g 4'-Cyano-4-hydroxybiphenyl werden in 250 ml Chloroform bei Siedehitze gelöst. Zu dieser Lösung wird unter weiterem Rückflußkochen 1 ml Brom in 20 ml Chloroform getropft. Die farblose Lösung wird abgekühlt und eingedampft.
Ausbeute: 5,48 g (100 % der Theorie),
Schmelzpunkt: 186-189°C
$R_f$-Wert: 0,66 (Kieselgel; 1,2-Dichlorethan/Essigester = 9:1)

| Ber.: | C 56,96 | H 2,94 | N 5,11 | Br 29,15 |
|---|---|---|---|---|
| Gef.: | 57,07 | 3,15 | 5,03 | 29,14 |

Beispiel B

(S)-1-(Benzyloxycarbonyl)-5-[(trityloxy)methyl]-2-pyrrolidinon

Eine Lösung von 160 g (S)-5-[(Trityloxy)methyl]-2-pyrrolidinon in 1600 ml trockenem Tetrahydrofuran wird innerhalb von 35 Minuten bei -65°C mit 179 ml einer 2,5-molaren Lösung von Butyllithium in Hexan versetzt. Nach 10 Minuten wird bei -65°C eine Lösung von 66,8 ml Chlorameisensäure-benzylester in 100 ml trockenem Tetrahydrofuran zugetropft und eine Stunde gerührt. Dann wird mit 200 ml gesättigter Kochsalzlösung versetzt und das Tetrahydrofuran abrotiert. Der Rückstand wird zwischen 3,5 l Essigester und 200 ml Wasser verteilt, die organische Phase abgetrennt und je zweimal mit Wasser und Kochsalzlösung gewaschen. Die organische Phase wird abgetrennt, getrocknet und einrotiert. Das Rohprodukt wird aus wenig Ethanol umkristallisiert.
Ausbeute: 181 g (82 % der Theorie),
Schmelzpunkt: 103-105°C $R_f$-Wert: 0,53 (Kieselgel; Cyclohexan/Essigester = 2:1)

| Ber.: | C 78,19 | H 5,95 | N 2,85 |
|-------|---------|--------|--------|
| Gef.: | 78,34 | 6,00 | 3,10 |

Beispiel C (35,5S)-1-(Benzyloxycarbonyl)-3-[(methoxycarbonyl)methyl]-5-[(trityloxy)methyl]-2-pyrrolidinon

Zu einer Lösung von 40,0 g (S)-1-(Benzyloxycarbonyl)-5-[(trityloxy)methyl]-2-pyrrolidinon in 400 ml wasserfreiem Tetrahydrofuran tropft man bei -65°C innerhalb von 20 Minuten unter Rühren 81,3 ml einer 1-molaren Lösung von Lithiumhexamethyldisilazid in Tetrahydrofuran. Nach 10-minütigem Rühren bei dieser Temperatur tropft man innerhalb von 30 Minuten eine Lösung von 7,5 ml Bromessigsäuremethylester in 50 ml wasserfreiem Tetrahydrofuran zu. Nach weiterem 45-minütigem Rühren bei -65°C läßt man die Reaktionslösung auf 0°C erwärmen und gibt 20 ml gesättigte Kochsalzlösung zu. Das Lösungsmittel wird im Vakuum abgedampft und der verbleibende Rückstand in 750 ml Essigester aufgenommen. Die organische Phase wird dreimal mit Wasser und einmal mit gesättigter Kochsalzlösung extrahiert, über Magnesiumsulfat getrocknet und im Vakuum eingeengt. Das Rohprodukt wird mit Cyclohexan/Essigester (2:1) über Kieselgel chromatographiert.
Ausbeute: 31,8 g (70 % der Theorie),
$R_f$-Wert: 0,54 (Kieselgel; Cyclohexan/Essigester = 2:1)

| Ber.: | C 74,58 | H 5,90 | N 2,49 |
|-------|---------|--------|--------|
| Gef.: | 74,61 | 6,09 | 2,43 |

Beispiel D

(3S,5S)-5-Hydroxymethyl-3-[(methoxycarbonyl)methyl]-2-pyrrolidinon

70,6 g (3S,5S)-1-(Benzyloxycarbonyl)-3-[(methoxycarbonyl)methyl]-5-[(trityloxy)methyl]-2-pyrrolidinon in 700 ml Methanol werden 3,5 Stunden bei 50°C unter einem Wasserstoffdruck von 5 bar mit 7 g eines Katalysators von 10 % Palladium auf Aktivkohle hydriert. Anschließend wird der Katalysator abfiltriert und das Filtrat eingeengt. Der Rückstand wird zweimal mit 150 ml Petrolether verrührt und die Petrolether-Phase abdekantiert. Das verbleibende Öl wird mit Methylenchlorid/Methanol (10:1) über Kieselgel chromatographiert.
Ausbeute: 17,0 g gelbliches Öl (73 % der Theorie),
$R_f$-Wert: 0,36 (Kieselgel; Methylenchlorid/Methanol = 10:1)

Beispiel E

(3S,5S)-5-[(Methansulfonyloxy)methyl]-3-[(methoxycarbonyl)methyl]-2-pyrrolidinon

Zu einer Lösung von 8,0 g (3S,5S)-5-Hydroxymethyl-3-[(methoxycarbonyl)methyl]-2-pyrrolidinon und 4,95 ml Methansulfonsäurechlorid in 100 ml Methylenchlorid tropft man bei 0°C innerhalb von 15 Minuten eine Lösung von 9,0 ml Triethylamin in 15 ml Methylenchlorid. Man rührt eine Stunde bei 0°C und eine weitere Stunde bei Raumtemperatur. Nach der Zugabe von 20 ml Wasser wird die organische Phase abgetrennt und zweimal mit Wasser gewaschen. Die organische Phase wird über Magnesiumsulfat getrocknet und im Vakuum eingeengt. Das Rohprodukt wird mit Essigester/Methanol (15:1) über Kieselgel chromatographiert und der erhaltene Feststoff aus Methyltert.butylether/Aceton umkristallisiert.
Ausbeute: 6,8 g (60 % der Theorie),
Schmelzpunkt: 85-87°C
$R_f$-Wert: 0,42 (Kieselgel; Aceton/Petrolether = 4:1)

| Ber.: | C 40,75 | H 5,70 | N 5,28 | S 12,09 |
|-------|---------|--------|--------|---------|
| Gef.: | 40,63 | 5,50 | 5,45 | 12,01 |

Beispiel F

4:1-Gemisch von (3S,5S)- und (3R,5S)-5-[(3-Brom-4'-cyano-4-biphenylyl)oxymethyl]-3-[(methoxycarbonyl)methyl]-2-pyrrolidinon

Eine Suspension von 7,0 g 3-Brom-4'-cyano-4-hydroxy-biphenyl und 10,5 g Cäsiumcarbonat in 150 ml Dimethylformamid wird eine Stunde bei Raumtemperatur gerührt. Nach Zugabe von 7,6 g (3S,5S)-5-[(Methansulfonyloxy)methyl]-3-[(methoxycarbonyl)methyl]-2-pyrrolidinon rührt man 2 Tage bei 55°C. Anschließend wird das Lösungsmittel weitgehend eingedampft und der Rückstand mit Kochsalzlösung und verdünnter Salzsäure versetzt. Man extrahiert die wässrige Phase mit Essigester, trocknet die organische Phase über Natriumsulfat und dampft ein. Das Rohprodukt wird mit Essigester über Kieselgel chromatographiert.
Ausbeute: 7,4 g (65 % der Theorie),
$R_f$-Wert: 0,51 (Kieselgel; Methylenchlorid/Methanol = 15:1)

| Ber.: | C 56,90 | H 4,32 | N 6,32 | Br 18,03 |
|---|---|---|---|---|
| Gef.: | 56,58 | 4,41 | 6,17 | 17,92 |

Beispiel G

(3S,5S)-5-[(4'-Amidino-3-brom-4-biphenylyl)oxymethyl]-3-[(methoxycarbonyl)methyl]-2-pyrrolidinon-hydrochlorid

In eine Lösung von 6,4 g des 4:1-Gemisches von (3S,5S)- und (3R,5S)-5-[(3-Brom-4'-cyano-4-biphenylyl)oxymethyl]-3-[(methoxycarbonyl)methyl]-2-pyrrolidinon in 250 ml wasserfreiem Methanol leitet man bei 0°C unter Rühren 2 Stunden Chlorwasserstoff ein. Nach 2-stündigem Rühren bei Raumtemperatur wird das Lösungsmittel im Vakuum bei einer Badtemperatur von 30°C abgedampft. Der Rückstand wird in 250 ml wasserfreiem Methanol gelöst und nach Zugabe von 20 g Ammoniumcarbonat 16 Stunden bei Raumtemperatur gerührt. Die Suspension wird über wenig Kieselgel filtriert und das Filtrat im Vakuum eingeengt. Das Rohprodukt wird mit Methylenchlorid/Methanol/konz. Ammoniak (4:1:0,25) über Kieselgel chromatographiert. Man erhält als letzte Fraktion das isomerenreine Produkt als Hydrochlorid.
Ausbeute: 270 mg (4 % der Theorie),
$R_f$-Wert: 0,16 (Kieselgel; Methylenchlorid/Methanol/konz. Ammoniak = 4:1:0,25)

Beispiel H

(3S,5S)-5-[[4-(5-Amidino-2-pyridyl)phenyl]oxymethyl]-3-[(tert.butyloxycarbonyl)methyl]-2-pyrrolidinon-hydrochlorid

2,1 g (3S,5S)-3-[(tert.Butyloxycarbonyl)methyl]-5-[[4-(5-cyano-2-pyridyl)phenyl]oxymethyl]-2-pyrrolidinon in 50 ml trockenem Methanol werden mit 8,5 ml 0,13 M Natriummethylat-Lösung 40 Stunden bei Raumtemperatur gerührt. Es werden 63 µl Eisessig und dann 0,5 g Ammoniumchlorid zugegeben und 2 1/2 Tage bei Raumtemperatur gerührt. Nach dem Einengen wird durch Säulenchromatographie auf Kieselgel mit Methylenchlorid/Methanol (6:1) gereinigt.
Ausbeute: 1 g (42 % der Theorie),
Schmelzpunkt: 207°C (Zers.)
$R_f$-Wert: 0,56 (Kieselgel; Methylenchlorid/Methanol = 4:1)
Analog wird folgende Verbindung erhalten:

(3S,5S)-5-[[4-(5-Amidino-2-pyrimidyl)phenyl]oxymethyl]-3-[(tert.butyloxycarbonyl)methyl]-2-pyrrolidinon-hydrochlorid

Schmelzpunkt: 277-279°C (Zers.)
$R_f$-Wert: 0,38 (reversed phase Kieselgel; Methanol/5%ige wäßrige Kochsalzlösung = 6:4)

Beispiel I

(3S,5S)-5-[(4'-Amidino-4-biphenylyl)oxymethyl]-3-[(methoxycarbonyl)methyl]-l-(3-phenylpropyl)-2-pyrrolidinon-hydrochloridsemihydrat

140 g (3S,5S)-5-[(4'-Cyano-4-biphenylyl)oxymethyl]-3-[(methoxycarbonyl)methyl]-l-(3-phenylpropyl)-2-pyrrolidinon werden in 1100 ml Methanol gelöst und auf -20°C abgekühlt. Man leitet bei dieser Temperatur 4 Stunden lang Salzsäuregas unter Rühren ein und rührt weitere 16 Stunden bei Raumtemperatur nach. Man dampft das Lösungsmittel im Vakuum ab, wobei das rohe Iminoester-hydrochlorid als zähes Öl zurück bleibt (172 g). Dieses Rohprodukt wird in 1500 ml Methanol gelöst und nach Zugabe von 144 g Ammoniumcarbonat 2 Stunden bei Raumtemperatur gerührt. Danach fügt man weitere 48 g Ammoniumcarbonat zu und rührt für weitere 1 1/2 Stunden. Das Reaktionsgemisch wird unter Rühren mit methanolischer Salzsäure auf pH 3,5 gebracht. Man engt nun im Vakuum auf etwa 800 ml ein und filtriert das ausgefallene Ammoniumchlorid ab. Das Filtrat wird nun bis zur beginnenden Kristallisation weiter eingeengt (auf etwa 350 ml). Nach beendeter Kristallisation filtriert man den Niederschlag ab und wäscht mit 75 ml eiskaltem Methanol und schließlich mit Aceton und Ether nach. Durch Einengen der Filtrate erhält man eine weitere Fraktion. Beide Kristallisate werden vereinigt und aus Methanol umkristallisiert.

Ausbeute: 128,7 g (83 % der Theorie),
Schmelzpunkt: 184-187°C (Zers.)

| Ber.: | C 66,11 | H 6,47 | N 7,71 | Cl 6,50 |
|-------|---------|--------|--------|---------|
| Gef.: | 65,98 | 6,41 | 7,67 | 6,67 |

Analog werden erhalten:

(1)   (3S,5S)-5-[(4'-Amidino-4-biphenylyl)oxymethyl]-3-[(methoxycarbonyl)methyl]-2-pyrrolidinon   x   1,25   HCl
Schmelzpunkt: ab 141°C (Zers.)
$R_f$-Wert: 0,30 (Kieselgel; Methylenchlorid/Methanol = 85:15)

| Ber.: | C 59,07 | H 5,72 | N 9.84 | Cl 10,38 |
|-------|---------|--------|--------|----------|
| Gef.: | 58,96 | 5,96 | 9,68 | 10,10 |

(2)  (3S,5S)-1-Acetyl-5-[(4'-amidino-4-biphenylyl)oxymethyl]-3-[(methoxycarbonyl)methyl]-pyrrolidin-hydrochlorid
$R_f$-Wert: 0,16 (Kieselgel; Methylenchlorid/Methanol = 10:1)

| Ber.: | C 61,95 | H 6,33 | N 9,42 | Cl 7,95 |
|-------|---------|--------|--------|---------|
| Gef.: | 61,76 | 6,31 | 9,11 | 7,84 |

(3)  (3R,5R)-5-[(4'-Amidino-4-biphenylyl)oxymethyl]-3-[(methoxycarbonyl)methyl]-2-pyrrolidinon-hydrochlorid-semihydrat Schmelzpunkt: 138°C (Zers.)
$R_f$-Wert: 0,52 (reversed phase Kieselgel (RP8); Methanol/ 10 % wäßrige Kochsalzlösung = 6:4)

| Ber.: | C 59,08 | H 5,90 | N 9,84 | Cl 8,31 |
|-------|---------|--------|--------|---------|
| Gef.: | 58,96 | 6,19 | 9,68 | 8,93 |

(4)    (3S,5S)-5-[(4'-Amidino-4-biphenylyl)oxymethyl]-3-[(methoxycarbonyl)methyl]-l-[(pyrrolidin-N-carbonyl)methyl]-2-pyrrolidinon-hydrochlorid
$R_f$-Wert: 0,46 (Kieselgel; Methylenchlorid/Methanol/konz. wäßriges Ammoniak = 30:10:2)
(5) (3S,5S)-5-[(4'-Amidino-4-biphenylyl)oxymethyl]-1-methan-sulfonyl-3-[(methoxycarbonyl)methyl]-pyrrolidin-hydrochlorid
$R_f$-Wert: 0,24 (Kieselgel; Methylenchlorid/Methanol = 10:1)

| Ber.: | C 54,82 | H 5,85 | N 8,72 | Cl 7,36 |
|-------|---------|--------|--------|---------|
| Gef.: | 54,68 | 5,82 | 8,47 | 7,20 |

Beispiel J

1-[6-(4-Amidino-phenyl)-3-pyridazinyl]-4-(2-methoxycarbonylethyl)-imidazol

Eine Mischung aus 1,1 g 1-[6-(4-Cyan-phenyl)-3-pyridazinyl]-4-(2-methoxycarbonyl-ethyl)-imidazol, 1500 ml absolutem Methanol und 50 ml Methylenchlorid wird unter Rühren und Eiskühlung mit trockenem Chlorwasserstoff gesättigt. Man rührt weitere 16 Stunden bei Raumtemperatur und destilliert das Lösungsmittel im Vakuum ab. Der Rückstand wird in 250 ml absolutem Methanol aufgenommen und mit 8 g Ammoniumcarbonat versetzt. Man rührt 30 Minuten bei Raumtemperatur, saugt den Niederschlag ab und dampft das Filtrat im Vakuum ein. Der Eindampfrückstand wird mit dem vorher gewonnenen Niederschlag vereinigt und säulenchromatographisch gereinigt (Elutionsmittel: Methylenchlorid/Methanol/konz. Ammoniak = 2:1:0,25).
Ausbeute: 0,36 g (31 % der Theorie),
$R_f$-Wert: 0,22 (Kieselgel; Methylenchlorid/Methanol/konz. Ammoniak = 2:1:0,25)
Analog werden folgende Verbindungen erhalten:

(1) 1-[6-(4-Amidino-phenyl)-3-pyridazinyl]-4-(2-hydroxy-2-methoxycarbonyl-ethyl)-imidazol
$R_f$-Wert: 0,17 (Kieselgel; Methylenchlorid/Methanol/konz. Ammoniak = 2:1:0,25)
(2) 1-[6-(4-Amidino-phenyl)-3-pyridazinyl]-4-(2-amino-2-methoxycarbonyl-ethyl)-imidazol-tris-trifluoracetat Als Ausgangsprodukt dient 1-[6-(4-Cyan-phenyl)-3-pyridazinyl]-4-(2-tert.butyloxycarbonylamino-2-methoxycarbonyl-lethyl)-imidazol
Die rohe freie Base wird durch Aufnehmen in Methylenchlorid, Versetzen mit Trifluoressigsäure, Einengen und Reinigen über Kieselgel (Elutionsmittel: Methylenchlorid/Methanol/konz. Ammoniak = 2:1:0,25) in das Tris-trifluoracetat überführt.
$R_f$-Wert: 0,18 (Kieselgel; Methylenchlorid/Methanol/konz. Ammoniak = 2:1:0,25)


Beispiel K


4-Amidino-4'-(5-methoxycarbonyl-pentyloxy)-biphenyl x 0,5 $H_2CO_3$


Man überschichtet 75 ml Methanol mit 30 ml Petroläther und leitet unter Eiskühlung Chlorwasserstoffgas bis zur Sättigung ein. Man trägt nun 2,1 g 4-Cyano-4'-(5-ethoxycarbonylpentyloxy)-biphenyl ein und rührt 18 Stunden bei Raumtemperatur. Man engt im Vakuum zur Trockene ein, suspendiert den Rückstand in Methanol, setzt 5,36 g Ammoniumcarbonat zu und rührt 16 Stunden bei Raumtemperatur. Der erhaltene Niederschlag wird abfiltriert und durch Verrühren mit Methylenchlorid/Methanol (85:15) und Wasser gereinigt.
Ausbeute: 1,75 g (75 % der Theorie),
Schmelzpunkt: 185-189°C (Zers.)

| Ber. (x 0,5 $H_2CO_3$): | C 66,31 | H 6,74 | N 6,55 |
|---|---|---|---|
| Gef.: | 66,75 | 6,85 | 7,41 |


Analog werden folgende Verbindungen erhalten:


(1) 4-Amidino-4'-[(4-methoxycarbonylmethyl-piperidino)carbonyl]-biphenyl-hydrochlorid Schmelzpunkt: 268-270°C
(2) 4-Amidino-4'-[(4-methoxycarbonylmethyl-piperidino)methyl]-biphenyl-hydrochlorid Schmelzpunkt: 148-150°C (Zers.)
(3) 4-Amidino-4'-[(4-methoxycarbonyl-cyclohexyl)-aminocarbonyl]-biphenyl-hydrochlorid Schmelzpunkt: 302-305°C (Zers.)
(4) 4-Amidino-4'-methoxy-3'-[(4-methoxycarbonyl-cyclohexyl)aminocarbonyl]-biphenyl $R_f$-Wert: 0,15 (Kieselgel; Methylenchlorid/Methanol = 9:1)
(5) 4-Amidino-4'-[N-(4-methoxycarbonyl-cyclohexyl)-N-methylaminocarbonyl]-biphenyl-hydrochlorid Schmelzpunkt: 295-300°C


Beispiel L


1-(4'-Amidino-4-biphenylyl)-3-methoxycarbonylmethyl-imidazolidin-2-on-hydrochlorid


Zu 3,1 g 1-(4'-Cyano-4-biphenylyl)-3-methoxycarbonylmethylimidazolidin-2-on gibt man 85 ml einer unter Eiskühlung gesättigten Lösung von Chlorwasserstoff in Methanol. Die entstandene Suspension wird mit Petrolether überschichtet und 3,5 Stunden bei Raumtemperatur gerührt. Man engt zur Trockene ein und trocknet noch 15 Minuten bei 1 mbar nach. Der Rückstand wird in 80 ml absolutem Methanol suspendiert, mit 2,7 g Ammoniumcarbonat versetzt und 16 Stunden bei Raumtemperatur gerührt. Man filtriert vom Niederschlag ab, engt die Mutterlauge ein und reinigt den Rückstand durch Säulenchromatographie an Kieselgel (Elutionsmittel: Methylenchlorid/ Methanol/konz. Ammoniak = 3:1:0,2).
Ausbeute: 0,7 g (20 % der Theorie),
Schmelzpunkt: über 200°C

R$_f$-Wert: 0,53 (Kieselgel; Methylenchlorid/Methanol/konz. Ammoniak = 3:1:0,2)

Analog werden folgende Verbindungen erhalten:

(1)   1-(4-Amidino-phenyl)-3-[4-(2-methoxycarbonyl-ethyl)cyclohexyl]-imidazolidin-2-on-hydrochlorid   Schmelzpunkt: über 200°C

R$_f$-Wert: 0,44 (Reversed Phase Platte RP8; Methanol/5%ige Natriumchloridlösung = 6:4)

(2)   1-(4-Amidino-phenyl)-3-[4-(2-methoxycarbonyl-ethyl)-phenyl]-imidazolidin-2,4-dion-hydrochlorid   Schmelzpunkt: über 260°C

R$_f$-Wert: 0,19 (Kieselgel; Methylenchlorid/Methanol = 9:1)

| Ber.: | C 57,62 | H 5,08 | N 13,44 | Cl 8,50 |
|---|---|---|---|---|
| Gef.: | 56,94 | 5,03 | 13,33 | 8,99 |

(3)   2-(4-Amidino-phenyl)-5-[4-(2-methoxycarbonyl-ethyl)-phenyl]-1,2,5-thiadiazolidin-1,1-dioxid-hydrochlorid Schmelzpunkt: 245-248°C (Zers.)

R$_f$-Wert: 0,44 (Reversed Phase Platte RP8; Methanol/10%ige Natriumchloridlösung = 6:4)

(4) 1-(4-Amidino-phenyl)-3-[4-(2-methoxycarbonyl-ethyl)phenyl]-4-methyl-imidazolin-2-on-hydrochlorid Schmelzpunkt: 248°C (Zers.)

R$_f$-Wert: 0,40 (Reversed Phase Platte RP8; Methanol/5%ige Natriumchloridlösung = 6:4)

(5)   2-(4-Amidino-phenyl)-4-[4-(2-methoxycarbonyl-ethyl)phenyl]-5-methyl-1,2,4-triazol-5-in-3-on-hydrochlorid Schmelzpunkt: 272-274°C

R$_f$-Wert: 0,37 (Reversed Phase Platte RP8; Methanol/5%ige Natriumchloridlösung = 6:4)

(6)   2-(4-Amidino-phenyl)-5-ethyl-4-[4-(2-methoxycarbonylethyl)-phenyl]-1,2,4-triazol-5-in-3-on-hydrochlorid Schmelzpunkt: über 250°C

R$_f$-Wert: 0,36 (Reversed Phase Platte RP8; Methanol/10%ige Natriumchloridlösung = 6:4)

| Ber. x HCl: | C 58,67 | H 5,63 | N 16,29 | Cl 8,25 |
|---|---|---|---|---|
| Gef.: | 58,01 | 5,65 | 16,26 | 9,14 |

(7)   2-(4-Amidino-phenyl)-4-[4-(2-methoxycarbonyl-ethyl)phenyl]-1,2,4-triazol-5-in-3-on-hydrochlorid   Schmelzpunkt: 275-277°C

R$_f$-Wert: 0,55 (Reversed Phase Platte RP8; Methanol/5%ige Natriumchloridlösung = 6:4)

(8)   4-(4-Amidino-phenyl)-2-[4-(2-methoxycarbonyl-ethyl)phenyl]-1,2,4-triazol-5-in-3-on-hydrochlorid   Schmelzpunkt: 289-291°C (Zers.)

R$_f$-Wert: 0,49 (Reversed Phase Platte RP8; Methanol/5%ige Natriumchloridlösung = 6:4)

Beispiel M

2-(4-Amidino-phenyl)-5-[(3-methoxycarbonyl-propyl)-aminocarbonyl]-1-methyl-benzimidazol-hydrochlorid

4,2 g 4-[4-[N-(4-Amidino-benzoyl)-methylamino]-3-nitro-benzoylamino]-buttersäure-methylester werden in 100 ml Methanol gelöst, mit 10 ml etherischer Salzsäure und 0,5 g 10%iger Palladiumkohle versetzt und bei Raumtemperatur mit Wasserstoff von 5 bar Druck 22 Stunden behandelt. Man setzt weitere 0,3 g des Katalysators zu und läßt eine weitere Stunde reagieren. Der Katalysator wird abfiltriert, das Filtrat eingedampft und der Rückstand mit einer Mischung aus 100 ml Essigester und 10 ml Methanol eine Stunde bei Raumtemperatur verrührt, wobei die Substanz in kristalliner Form anfällt.

Ausbeute: 3,7 g (100 % der Theorie),

Schmelzpunkt: über 200°C

R$_f$-Wert: 0,65 (Reversed-Phase-Platte RP18; Methanol/5%ige wäßrige Natriumchloridlösung = 6:4)

Analog wird folgende Verbindung erhalten:

(1) 2-(4-Amidino-phenyl)-5-[(2-methoxycarbonyl-ethyl)-aminocarbonyl]-1-methyl-benzimidazol

R$_f$-Wert: 0,59 (Reversed-Phase-Platte RP18; Methanol/5%ige wäßrige Natriumchloridlösung = 6:4)

Beispiel N

2-[(4-Amidino-phenyl)-oxymethyl]-5(6)-methoxycarbonylmethoxy-benzimidazol-hydrochlorid

2,4 g 2-[(4-Cyan-phenyl)-oxymethyl]-5(6)-methoxycarbonylmethoxy-benzimidazol werden in 300 ml Methanol suspendiert. In die Mischung leitet man eine Stunde lang bei 0-10°C Salzsäuregas ein und rührt 4 Stunden bei 15-20°C nach. Das Methanol wird im Vakuum abgedampft, der Rückstand mit 75 ml Methanol versetzt und dieses wiederum im Vakuum abdestilliert. Der Rückstand wird in 300 ml Methanol suspendiert, wonach man unter Rühren portionsweise 16,3 g Ammoniumcarbonat zufügt und weitere 16 Stunden nachrührt. Die Reaktionsmischung wird mit einer Mischung aus 3 Teilen Methanol und einem Teil konzentrierter Salzsäure auf pH4 gebracht. Man dampft zur Trockene ein und reinigt den Rückstand über Kieselgel (Elutionsmittel: Methylenchlorid/Methanol = 3:1 bis 0:1).
Ausbeute: 0,9 g (32 % der Theorie),
Schmelzpunkt: 250°C (Zers.)
$R_f$-Wert: 0,64 (Kieselgel; Methylenchlorid/Methanol/Eisessig = 3:1:0,1)

| Ber. x $H_2O$ x HCl: | C 52,87 | H 5,18 | N 13,70 | Cl 8,67 |
| --- | --- | --- | --- | --- |
| Gef.: | 53,07 | 5,02 | 13,81 | 8,80 |

Analog werden folgende Verbindungen erhalten:

(1) 2-(4-Amidino-phenyl)-1-[2-(3,4-dimethoxy-phenyl)-ethyl]-5-[(2-methoxycarbonyl-ethyl)-aminocarbonyl]-benzimidazol-hydrochlorid
$R_f$-Wert: 0,20 (Kieselgel; Essigester/Ethanol = 7:3)
(2) 2-(4-Amidino-phenyl)-5-[(2-methoxycarbonyl-ethyl)-aminocarbonyl]-1-(2-piperazino-ethyl]-benzimidazol
Schmelzpunkt: 80°C (Zers.)
$R_f$-Wert: 0,14 (Kieselgel; Isopropanol/Wasser/konz. Ammoniak = 7:2:1, nach zweimaliger Entwicklung)
(3) 2-(4-Amidino-phenyl)-5-[(2-methoxycarbonyl-ethyl)-aminocarbonyl]-1-(3-thiomorpholino-propyl)-benzimidazol
$R_f$-Wert: 0,18 (Kieselgel; Methylenchlorid/Methanol/konz. Ammoniak = 8:2:0,1)

Herstellung der Endprodukte:

Beispiel 1

(3S,5S)-5-[(4'-Amidino-4-biphenylyl)oxymethyl]-3-[(methoxycarbonyl)methyl]-2-pyrrolidinon-hydrochlorid[3-$^3$H-4-biphenylyl]

In einer geschlossenen Apparatur wird eine Lösung von 25 mg (3S,5S)-5-[(4'-Amidino-3-brom-4-biphenylyl)oxymethyl]-3-[(methoxycarbonyl)methyl]-2-pyrrolidinon-hydrochlorid in 0,5 ml Dimethylformamid in Gegenwart von 10 mg eines Katalysators von 10 % Palladium auf Aktivkohle unter kräftigem Rühren mit 370 GBq trägerfreiem Tritiumgas behandelt. Nach 4 Stunden ist die Aufnahme von Tritium beendet. Die Reaktionslösung wird mit weiterem Dimethylformamid verdünnt, der Katalysator abfiltriert und das Lösungsmittel unter vermindertem Druck bei 80°C abgezogen. Bei diesem Schritt wird der Großteil des labilen Tritiums entfernt. Zur Entfernung des restlichen labilen Tritiums wird der Rückstand in 50 ml absolutem Ethanol aufgenommen, 3 Tage bei Raumtemperatur stehengelassen und dann bei 35°C bei vermindertem Druck das Ethanol wieder vollständig abgezogen. Der Rückstand wird zur Lagerung in 50 ml absolutem Ethanol aufgenommen.

Radiochemische Ausbeute: 13,14 GBq (3,6 % der Theorie bezogen auf eingesetzte Tritium-Gesamtaktivität)
Radiochemische Reinheit: 98,8 % der Theorie
Bestimmungsmethode HPLC: Säule 4 x 125 mm Kromasil 100, C 18, 5 μm
Fluß 1,5 ml/Minute
Fließmittel A 0,1 % $KH_2PO_4$, pH 2,5 ($H_3PO_4$)
Fließmittel B Methanol
Gradient in 10 Minuten von 95 % A nach
65 % A, weiter 20 Minuten 65 % A

Beispiel 2

(3S,5S)-5-[(4'-Amidino-4-biphenylyl)oxymethyl]-3-[(carboxyl)methyl]-2-pyrrolidinon[3-$^3$H-4-biphenylyl]

Eine Lösung von 2,57 mg (1,577 GBq) (3S,5S)-5-[(4'-Amidino-4-biphenylyl)oxymethyl]-3-[(methoxycarbonyl)-methyl]-2-pyrrolidinon-[3-3H-4-biphenylyl] in 6 ml Ethanol wird bei vermindertem Druck bei 35°C eingeengt. Den Rückstand löst man in 300 µl Methanol und setzt 50 µl 2N Natronlauge zu (pH 12). Nach 12 Stunden bei Raumtemperatur wird erst mit 600 µl Wasser versetzt und dann Ammoniumchlorid bis zum pH 8 zugegeben. Anschließend werden nochmals 180 µl Wasser und 60 µl Methanol zugegeben und diese klare Lösung zur Reinigung auf RP-8 Dünnschicht-Fertigplatten (Fa. Merck; Fließmittel: 60 % Methanol/40 % 10%ige Kochsalzlösung) chromatographiert. Die im UV-Licht (254 und 366 nm) gut erkennbaren Bahnen mit der Titelverbindung werden unter den üblichen Vorsichtsmaßnahmen von den noch feuchten Platten abgekratzt, mit 4 ml Dimethylsulfoxid und 200 µl 1N Salzsäure gründlich ausgerührt. Die Suspension wird über ein Membranfilter, Porenweite 0,5 µm, filtriert. Man erhält 3,5 ml Lösung der [$^3$H]Titelverbindung.

Radiochemische Reinheit:  97,8 % der Theorie

| Bestimmungsmethode HPLC: | Säule | 4 x 125 mm Kromasil 100, C 18, 5 µm |
| | Fluß | 1,5 ml/Minute |
| | Fließmittel A | 0,1 % KH$_2$PO$_4$, pH 2,5 (H$_3$PO$_4$) |
| | Fließmittel B | Methanol |
| | Gradient | in 10 Minuten von 95 % A nach 65 % A, weiter 20 Minuten 65 % A |

Identität:  Durch HPLC im Vergleich mit inaktivem, analysenreinem Material nachgewiesen.
Gehaltsbestimmung (UV-spektrophotometrisch):0,371 mg/ml
Aktivitätskonzentration (Flüssigscintillationsmethode):258,5 MBq/ml
Spezifische Aktivität:256 GBq/mMol = 0,697 MBq/mg
Chemische Ausbeute:1,298 mg (57 % der Theorie)

Beispiel 3

(3S,5S)-5-[(4'-Amidino-4-biphenylyl)oxymethyl]-3-[(carboxy)methyl]-2-pyrrolidinon[3-$^3$H-4-biphenylyl]

Eine Lösung von 2,14 mg (3S,5S)-5-[(4'-Amidino-4-biphenylyl)oxymethyl]-3-[(methoxycarbonyl)methyl]-2-pyrroli-dinon-hydrochlorid[3-$^3$H-4-biphenylyl] in 5 ml Ethanol mit einer Gesamtradioaktivität von 1,314 GBq wird bei vermindertem Druck bei 35°C eingeengt. Den Rückstand löst man in 350 µl Methanol und setzt 25 µl 2N Natronlauge zu (pH 12). Nach 12 Stunden bei Raumtemperatur werden die ausgefallenen Kristalle auf den Boden des Reaktionsgefäßes zentrifugiert und der klare Überstand abpipettiert. Der kristalline Rückstand wird 3 mal mit je 100 µl Methanol gewaschen, in 300 µl Dimethylsulfoxid und 40 µl 1N Salzsäure gelöst und anschließend mit weiterem Dimethylsulfoxid auf ein Gesamtvolumen von 3 ml gebracht.

Radiochemische Ausbeute:  425 MBq (32 % der Theorie)
Chemische Ausbeute:  0,61 mg (32 % der Theorie)
Identität:  Durch HPLC im Vergleich mit inaktivem, analysenreinem Material nachgewiesen.

**Patentansprüche**

1. Verwendung eines Fibrinogen-Rezeptor-Antagonisten, der eine vergleichbare oder höhere Affinität gegenüber dem Rezeptor als [125]J-Fibrinogen aufweist und in Gegenwart von Fremdprotein eine Affinität (K$_D$) von weniger als 500nM gegenüber dem Rezeptor aufweist sowie mindestens ein detektierbares Tritiumatom anstelle von Wasserstoff enthält, zur Bestimmung der Bindung von chemischen Substanzen an Fibrinogen-Rezeptoren.

2. Verwendung eines Fibrinogen-Rezeptor-Antagonisten, der eine vergleichbare oder höhere Affinität gegenüber dem Rezeptor als [125]J-Fibrinogen aufweist und in Gegenwart von Fremdprotein eine Affinität (K$_D$) von weniger als 500nM gegenüber dem Rezeptor aufweist sowie mindestens ein detektierbares Tritiumatom anstelle von Wasserstoff enthält, zur Konzentrationsbestimmung von Fibrinogen-Rezeptor-Antagonisten.

3. Verwendung eines Fibrinogen-Rezeptor-Antagonisten gemäß Anspruch 1 oder 2, dadurch gekennzeichnet, daß die Bestimmung in Gegenwart von Fremdeiweiß und/oder von Körperflüssigkeiten durchgeführt wird.

4. Verwendung eines Fibrinogen-Rezeptor-Antagonisten gemäß den Ansprüchen 1 oder 2, dadurch gekennzeichnet, daß als Fibrinogen-Rezeptor-Antagonist ein Amidin der allgemeinen Formel

$$R_a - R_b \qquad\qquad (I)$$

in der

$R_a$ eine 4-Amidinophenyl- oder 5-Amidino-pyrimid-2-yl-Gruppe und

$R_b$ eine HOOC-D-C-B-A-Gruppe bedeuten, in der

A eine gegebenenfalls durch eine Methoxygruppe substituierte Phenylengruppe, in der zusätzlich eine oder zwei Methingruppen jeweils durch ein Stickstoffatom ersetzt sein können, eine gegebenenfalls an einem Kohlenstoffatom durch eine Methyl-, Ethyl- oder Trifluormethylgruppe substituierte Imidazolinon-di-yl-, Imidazolidinon-di-yl-, Imidazolidin-dion-di-yl-, Triazolinon-di-yl- oder 1,1-Dioxo-1,2,5-thiadiazolidin-di-yl-gruppe, eine gegebenenfalls an einem der Stickstoffatome durch den Rest $R_1$ substituierte Benzimidazol-di-yl-gruppe oder eine mit dem Stickstoffatom an den Rest B gebundene Aminocarbonylgruppe,
B eine Methylen-, Carbonyl-, Cyclohexylen-, Phenylen- oder Imidazol-di-yl-gruppe, eine über das Stickstoffatom an den Rest C gebundene Aminocarbonylgruppe, welche gleichzeitig am Stickstoffatom durch eine Methylgruppe substituiert sein kann, oder eine über das Sauerstoffatom an den Rest A gebundene Methylenoxygruppe,
C eine gegebenenfalls durch den Rest $R_2$ substituierte Ethylengruppe, eine Cyclohexylengruppe, eine gegebenenfalls am Stickstoffatom durch den Rest $R_3$ substituierte Pyrrolidin-di-yl- oder Pyrrolidinon-di-yl-gruppe, eine Piperidin-di-yl-gruppe oder eine mit dem Stickstoffatom an den Rest D gebundene Aminocarbonylgruppe und
D eine Bindung, eine Methylen- oder Ethylengruppe darstellen, wobei

$R_1$ eine Methyl-, 2-Piperazinoethyl-, 2-(3,4-Dimethoxyphenyl)ethyl- oder 3-Thiomorpholinopropylgruppe,
$R_2$ eine Amino- oder Hydroxygruppe und
$R_3$ eine 3-Phenylpropyl-, Acetyl-, Methansulfonyl- oder Pyrrolidinocarbonylmethylgruppe darstellen,

deren Tautomere, deren Stereoisomere einschließlich deren Gemische und deren Salze eingesetzt wird, in dem mindestens ein Wasserstoffatom durch Tritium ersetzt ist.

5. Verwendung eines Fibrinogen-Rezeptor-Antagonisten gemäß den Ansprüchen 1 oder 2, dadurch gekennzeichnet, daß als Fibrinogen-Rezeptor-Antagonist ein Amidin der allgemeinen Formel I gemäß Anspruch 4, in dem

$R_a$ eine 4-Amidino-phenylgruppe oder auch, wenn $R_b$ eine in 4-Stellung durch eine 3-Carboxymethyl-pyrrolidin-2-on-5-yl-methyloxygruppe substituierte Phenylgruppe darstellt, eine 5-Amidino-pyrimid-2-yl-Gruppe und

$R_b$ eine Phenylgruppe, die in 4-Stellung durch eine 4-Carboxymethyl-pyrrolidin-2-yl-methyloxy-, 3-Carboxymethyl-pyrrolidin-2-on-5-yl-methyloxy-, 4-Carboxymethyl-piperidinomethyl-, 4-Carboxymethyl-piperidinocarbonyl-, 4-Carboxycyclohexylaminocarbonyl- oder N-Methyl-N-(4-carboxycyclohexyl)aminocarbonylgruppe substituiert ist, wobei jeweils in 1-Stellung der Pyrrolidinteil durch eine Acetyl- oder Methansulfonylgruppe und der Pyrrolidinonteil durch eine 3-Phenylpropyl- oder Pyrrolidinocarbonylmethylgruppe substituiert sein kann,

eine 4-Methoxy-phenylgruppe, die in 3-Stellung durch eine 4-Carboxycyclohexyl-aminocarbonylgruppe substituiert ist,
eine in 6-Stellung durch eine Imidazol-l-yl-Gruppe substituierte Pyridazin-3-yl-Gruppe, wobei der Imidazolylteil in 4-Stellung durch eine 2-Amino-2-carboxy-ethyl- oder 2-Carboxy-2-hydroxy-ethylgruppe substituiert ist,
eine Imidazolidin-2-on-1-yl-Gruppe, die in 3-Stellung durch eine 4-(2-Carboxyethyl)-cyclohexyl- oder 4-(2-Carboxyethyl)phenylgruppe substituiert ist,
eine 4-Methyl-4-imidazolin-2-on-1-yl- oder Imidazolidin-2,4-dion-1-yl-Gruppe, die in 3-Stellung durch eine 4-(2-Carboxyethyl)-phenyl-Gruppe substituiert ist,

eine gegebenenfalls in 5-Stellung durch eine Methyl- oder Ethylgruppe substituierte 1,2,4-Triazol-5-in-3-on-2-yl-Gruppe, die in 4-Stellung durch eine 4-(2-Carboxyethyl)-phenylgruppe substituiert ist,

eine 1,2,4-Triazol-5-in-3-on-4-yl-Gruppe, die in 2-Stellung durch eine 4-(2-Carboxyethyl)-phenyl-Gruppe substituiert ist,

eine in 5-Stellung durch eine 4-(2-Carboxyethyl)-phenylgruppe substituierte 1,1-Dioxo-1,2,5-thiadiazolidin-2-yl-Gruppe,

eine gegebenenfalls in 1-Stellung durch eine Methyl-, 2-Piperazino-ethyl- oder 2-(3,4-Dimethoxyphenyl)-ethylgruppe substituierte Benzimidazol-2-yl-Gruppe, die in 5-Stellung durch eine 2-Carboxyethylaminocarbonylgruppe substituiert ist, oder

eine Phenylaminocarbonylgruppe, die in 3-Stellung durch eine 2-Carboxyethylaminocarbonylgruppe substituiert ist, bedeuten,

deren Tautomere, deren Stereoisomere einschließlich deren Gemische und deren Salze eingesetzt wird, in dem mindestens ein Wasserstoffatom durch Tritium ersetzt ist.

6. Verwendung eines Fibrinogen-Rezeptor-Antagonisten gemäß den Ansprüchen 1 oder 2, dadurch gekennzeichnet, daß als Fibrinogen-Rezeptor-Antagonist eine der folgenden Verbindungen der allgemeinen Formel I gemäß Anspruch 4, in denen mindestens ein Wasserstoffatom durch Tritium ersetzt ist, eingesetzt wird:

(1) (3S,5S)- und (3R,5R)-5-[[(4-(5-Amidinopyrimid-2-yl)-phenyl]-oxymethyl]-3-carboxymethyl-pyrrolidin-2-on,
(2) (3S,5S)- und (3R,5R)-1-Acetyl-5-[(4'-amidino-4-biphenyl-yl)-oxymethyl]-3-carboxymethyl-pyrrolidin,
(3) (3S,5S)- und (3R,5R)-5-[(4'-Amidino-4-biphenylyl)-oxymethyl]-3-carboxymethyl-1-methansulfonyl-pyrrolidin,
(4) (3S,5S)- und (3R,5R)-5-[(4'-Amidino-4-biphenylyl)-oxymethyl]-3-carboxymethyl-pyrrolidin-2-on,
(5) 4-Amidino-4'-[(trans-4-carboxycyclohexyl)-aminocarbonyl]biphenyl,
(6) 4-Amidino-4'-[N-(trans-4-carboxycyclohexyl)-N-methylaminocarbonyl]-biphenyl,
(7) 1-(4-Amidinophenyl)-3-[4-(2-carboxyethyl)-phenyl]-imidazolidin-2,4-dion,
(8) 1-(4-Amidinophenyl)-3-[4-(2-carboxyethyl)-phenyl]-4-methyl-4-imidazolin-2-on,
(9) 2-(4-Amidinophenyl)-4-[4-(2-carboxyethyl)-phenyl]-1,2,4-triazol-5-in-3-on,
(10) 4-(4-Amidinophenyl)-2-[4-(2-carboxyethyl)-phenyl] triazol-5-in-3-on,
(11) 2-(4-Amidinophenyl)-4-[4-(2-carboxyethyl)-phenyl]-5-methyl-1,2,4-triazol-5-in-3-on,
(12) 2-(4-Amidinophenyl)-4-[4-(2-carboxyethyl)-phenyl]-5-ethyl-1,2,4-triazol-5-in-3-on,
(13) 2-(4-Amidinopheny1)-5-[4-(2-carboxyethyl)-phenyl]-1,2,5-thiadiazo1idin-1,1-dioxid,
(14) 1-(4-Amidinophenyl)-3-[4-(2-carboxyethyl)-cyclohexyl]imidazolidin-2-on,
(15) 2-(4-Amidinophenyl)-5-[(2-carboxyethyl)-aminocarbonyl]1-[2-(piperazin-1-yl)-ethyl]-benzimidazol,
(16) 4-Amidino-4'-[4-(carboxymethyl)-piperidinocarbonyl]-biphenyl und

deren Salze.

7. Verwendung eines Fibrinogen-Rezeptor-Antagonisten gemäß den Ansprüchen 1 oder 2, dadurch gekennzeichnet, daß als Fibrinogen-Rezeptor-Antagonist eine der folgenden Verbindungen der allgemeinen Formel I gemäß Anspruch 4, in denen mindestens ein Wasserstoffatom durch Tritium ersetzt ist, eingesetzt wird:

(1) (3S,5S)- und (3R,5R)-5-[[(4-(5-Amidinopyrimid-2-yl)-phenyl]-oxymethyl]-3-carboxymethyl-pyrrolidin-2-on,
(2) (3S,5S)- und (3R,5R)-1-Acetyl-5-[(4'-amidino-4-biphenyl-yl)-oxymethyl]-3-carboxymethyl-pyrrolidin,
(3) (3S,5S)- und (3R,5R)-5-[(4'-Amidino-4-biphenylyl)-oxymethyl]-3-carboxymethyl-1-methansulfonyl-pyrrolidin,
(4) (3S,5S)- und (3R,5R)-5-[(4'-Amidino-4-biphenylyl)-oxymethyl]-3-carboxymethyl-pyrrolidin-2-on,
(5) 4-Amidino-4'-[4-(carboxymethyl)-piperidinocarbonyl]-biphenyl und

deren Salze.

8. Verwendung eines Fibrinogen-Rezeptor-Antagonisten gemäß den Ansprüchen 1 oder 2, dadurch gekennzeichnet, daß in den Rezeptor-Antagonisten der allgemeinen Formel I gemäß den Ansprüchen 4 bis 7 ein aromatisches Wasserstoffatom durch Tritium ersetzt ist.

9. Verwendung eines Fibrinogen-Rezeptor-Antagonisten gemäß den Ansprüchen 1 oder 2, dadurch gekennzeichnet, daß als markierter Fibrinogen-Rezeptor-Antagonist die Verbindung (3S,5S)-5-[(4'-Amidino-4-biphenylyl)oxymethyl]-3-[(carboxy)methyl]-2-pyrrolidinon[3-$^3$H-4-biphenylyl] und dessen Salze eingesetzt wird.

10. Tritium markierte Fibrinogen-Rezeptor-Antagonisten der allgemeinen Formel I gemäß den Ansprüchen 4 bis 9, deren Tautomeren, deren Stereoisomere einschließlich deren Gemische und deren Salze.

11. Verfahren zur Herstellung der mit Tritium markierten Fibrinogen-Rezeptor-Antagonisten gemäß Anspruch 10, dadurch gekennzeichnet, daß in einem entsprechenden nicht-markierten Fibrinogen-Rezeptor-Antagonisten

      a) durch katalytische Tritiierung einer ungesättigten Kohlenstoff-Kohlenstoff-Bindung,
      b) durch hydrogenolytischen Austausch eines Halogenatoms,
      c) durch katalytischen Wasserstoff/Tritiumaustausch,
      d) durch katalytischen Wasserstoff/Tritiumaustausch in tritiierten Lösungsmitteln oder
      e) durch Tritiierung einer Vorstufe des herzustellenden Fibrinogen-Rezeptor-Antagonisten und anschließende Synthese des Fibrinogen-Rezeptor-Antagonisten

      mindestens ein Tritiumatom anstatt von Wasserstoff eingeführt wird und
      gewünschtenfalls anschließend ein so erhaltenes Isomerengemisch in seine Enantiomere aufgetrennt wird und/oder
      eine so erhaltene tritiierte Verbindung in ihr Salz übergeführt wird.

## Claims

1. Use of a fibrinogen receptor antagonist, which has an affinity for the receptor which is comparable to or greater than that of $^{125}$I-fibrinogen and in the presence of foreign protein has an affinity ($K_D$) of less than 500nM for the receptor and contains, instead of hydrogen, at least one detectable tritium atom, for determining the binding of chemical substances to fibrinogen receptors.

2. Use of a fibrinogen receptor antagonist, which has an affinity for the receptor which is comparable to or greater than that of $^{125}$I-fibrinogen and in the presence of foreign protein has an affinity ($K_D$) of less than 500 nM for the receptor and contains, instead of hydrogen, at least one detectable tritium atom, for determining the concentration of fibrinogen receptor antagonists.

3. Use of a fibrinogen receptor antagonist according to claim 1 or 2, characterised in that the measurement is carried out in the presence of foreign albumin and/or body fluids.

4. Use of a fibrinogen receptor antagonist according to claims 1 or 2, characterised in that the fibrinogen receptor antagonist used is an amidine of general Formula

$$R_a - R_b \qquad\qquad (I)$$

wherein

    $R_a$ denotes a 4-amidinophenyl or 5-amidino-pyrimid-2-yl group and
    $R_b$ denotes an HOOC-D-C-B-A- group, wherein

    A denotes an optionally methoxy-substituted phenylene group in which, additionally, one or two methine groups may be replaced by a nitrogen atom, or A denotes an imidazolinon-di-yl, imidazolidinondi-yl, imidazolidin-dion-di-yl, triazolinon-di-yl or 1,1-dioxo-1,2,5-thiadiazolidin-di-yl group each optionally substituted at a carbon atom by a methyl, ethyl or trifluoromethyl group, or A denotes a benzimidazol-di-yl group optionally substituted by the group $R_1$ at one of the nitrogen atoms, or A denotes an aminocarbonyl group bound to the group B by the nitrogen atom,
    B denotes a methylene, carbonyl, cyclohexylene, phenylene or imidazol-di-yl group, an aminocarbonyl group bound to the group C via the nitrogen atom, which may simultaneously be substituted by a methyl group at the nitrogen atom, or a methyleneoxy group bound to the group A via the oxygen atom,
    C denotes an ethylene group optionally substituted by the group $R_2$, a cyclohexylene group, a pyrrolidin-di-yl or pyrrolidinon-di-yl group optionally substituted by the group $R_3$ at the nitrogen atom, a piperidin-di-yl group or an aminocarbonyl group bound to the group D via the nitrogen atom and
D denotes a bond or a methylene or ethylene group, whilst

$R_1$ denotes a methyl, 2-piperazinoethyl, 2-(3,4-dimethoxyphenyl)ethyl or 3-thiomorpholinopropyl group,
$R_2$ denotes an amino or hydroxy group and
$R_3$ denotes a 3-phenylpropyl, acetyl,

methanesulphonyl or pyrrolidinocarbonylmethyl group, the tautomers, stereoisomers including the mixtures thereof and the salts thereof, in which at least one hydrogen atom is replaced by tritium.

5. Use of a fibrinogen receptor antagonist according to claim 1 or 2, characterised in that an amidine of general formula I according to claim 4 is used as fibrinogen receptor antagonist wherein

$R_a$ denotes a 4-amidino-phenyl group or, if $R_b$ denotes a phenyl group substituted in the 4-position by a 3-carboxymethyl-pyrrolidin-2-on-5-yl-methyloxy group, $R_a$ may also denote a 5-amidino-pyrimid-2-yl group and
$R_b$ denotes a phenyl group which is substituted in the 4-position by a 4-carboxymethyl-pyrrolidin-2-yl-methyloxy, 3-carboxymethyl-pyrrolidin-2-on-5-yl-methyloxy, 4-carboxymethyl-piperidinomethyl, 4-carboxymethylpiperdinocarbonyl, 4-carboxycyclohexylaminocarbonyl or N-methyl-N-(4-carboxycyclohexyl) -aminocarbonyl group, whilst in the 1-position each pyrrolidine moiety may be susbtituted by an acetyl or methanesulphonyl group and the pyrrolidinone moiety may be substituted by a 3-phenyl-propyl or pyrrolidinocarbonylmethyl group,

a 4-methoxy-phenyl group substituted in the 3-position by a 4-carboxycyclohexyl-aminocarbonyl group,

a pyridazin-3-yl group substituted in the 6-position by an imidazol-1-yl group, wherein the imidazolyl moiety is substituted in the 4-position by a 2-amino-2-carboxyethyl or 2-carboxy-2-hydroxy-ethyl group,

an imidazolidin-2-on-1-yl group substituted in the 3-position by a 4-(2-carboxyethyl)-cyclohexyl or 4-(2-carboxyethyl)-phenyl group,

a 4-methyl-4-imidazolin-2-on-1-yl or imidazolidin-2,4-dion-1-yl group substituted in the 3-position by a 4-(2-carboxy-ethyl)-phenyl group,

a 1,2,4-triazol-5-in-3-on-2-yl group optionally methyl- or ethyl-substituted in the 5-position and substituted in the 4-position by a 4-(2-carboxyethyl)-phenyl group,

a 1,2,4-triazol-5-in-3-on-4-yl group substituted in the 2-position by a 4-(2-carboxyethyl)-phenyl group,

a 1,1-dioxo-1,2,5-thiadiazolidin-2-yl group substituted in the 5-position by a 4-(2-carboxyethyl)-phenyl group,

a benzimidazol-2-yl group optionally substituted in the 1-position by a methyl, 2-piperazino-ethyl or 2-(3,4-dimethoxyphenyl)-ethyl group and substituted in the 5-position by a 2-carboxyethylaminocarbonyl group, or

a phenylaminocarbonyl group substituted in the 3-position by a 2-carboxyethylaminocarbonyl group,

the tautomers thereof, the stereoisomers including the mixtures thereof and the salts thereof, in which at least one hydrogen atom is replaced by tritium.

6. Use of a fibrinogen receptor antagonist according to claim 1 or 2, characterised in that one of the following compounds of general formula I according to claim 4, in which at least one hydrogen atom is replaced by tritium, is used as fibrinogen receptor antagonist:

(1) (3S,5S) - and (3R,5R)-5-[[(4-(5-amidinopyrimid-2-yl)phenyl]-oxymethyl]-3-carboxymethyl-pyrrolidin-2-one,

(2) (3S 5S) - and (3R,5R)-1-acetyl-5-[(4'-amidino-4-biphenylyl)-oxymethyl]-3-carboxymethyl-pyrrolidine,

(3) (3S,5S)- and (3R,5R)-5-[(4'-amidino-4-biphenylyl)oxymethyl]-3-carboxymethyl-1-methanesulphonylpyrrolidine,

(4) (3S,5S) - and (3R,5R)-5-[(4'-amidino-4-biphenylyl)oxymethyl]-3-carboxymethyl-pyrrolidin-2-one,

(5) 4-amidino-4'-[(trans-4-carboxycyclohexyl)aminocarbonyl]-biphenyl,

(6) 4-amidino-4'-[N-(trans-4-carboxycyclohexyl)-N-methyl-aminocarbonyl]-biphenyl,

(7) 1-(4-amidinophenyl)-3-[4-(2-carboxyethyl)-phenyl]imidazolidin-2,4-dione,

(8) 1-(4-amidinophenyl)-3-[4-(2-carboxyethyl)-phenyl]-4-methyl-4-imidazolin-2-one,

(9) 2-(4-amidinophenyl)-4-[4-(2-carboxyethyl)-phenyl]-1,2,4-triazol-5-in-3-one,

(10) 4-(4-amidinophenyl)-2-[4-(2-carboxyethyl)-phenyl]-1,2,4-triazol-5-in-3-one,

(11) 2-(4-amidinophenyl)-4-[4-(2-carboxyethyl)-phenyl]-5-methyl-1,2,4-triazol-5-in-3-one,

(12) 2-(4-Amidinophenyl)-4-[4-(2-carboxyethyl)-phenyl]-5-ethyl-1,2,4-triazol-5-in-3-one,

(13) 2-(4-amidinophenyl)-5-[4-(2-carboxyethyl)-phenyl]-1,2,5-thiadiazo1idin-1,1-dioxide,

(14) 1-(4-amidinophenyl)-3-[4-(2-carboxyethyl)cyclohexyl]-imidazolidin-2-one,

(15) 2-(4-amidinophenyl)-5-[(2-carboxyethyl)aminocarbonyl]-1-[2-(piperazin-1-yl)-ethyl]benzimidazole,

(16) 4-amidino-4'-[4-(carboxymethyl)-piperdinocarbonyl]biphenyl and

the salts thereof.

7. Use of a fibrinogen receptor antagonist according to claim 1 or 2, characterised in that one of the following compounds of general formula I according to claim 4, in which at least one hydrogen atom is replaced by tritium, is used as fibrinogen receptor antagonist:

(1) (3S,5S)- and (3R,5R)-5-[[(4-(5-amidinopyrimid-2-yl)phenyl]-oxymethyl]-3-carboxymethyl-pyrrolidin-2-one,

(2) (3S,5S)- and (3R,5R)-1-acetyl-5-[(4'-amidino-4-biphenylyl)-oxymethyl]-3-carboxymethyl-pyrrolidine,

(3) (3S,5S)- and (3R,5R)-5-[(4'-amidino-4-biphenylyl)oxymethyl]-3-carboxymethyl-1-methanesulphonylpyrrolidine,

(4) (3S,5S)- and (3R,5R)-5-[(4'-amidino-4-biphenylyl)oxymethyl]-3-carboxymethyl-pyrrolidin-2-one,

(5) 4-amidino-4'-[4-(carboxymethyl)-piperdinocarbonyl]biphenyl and

the salts thereof.

8. Use of a fibrinogen receptor antagonist according to claim 1 or 2, characterised in that an aromatic hydrogen atom is replaced by tritium in the receptor antagonists of general formula I according to claims 4 to 7.

9. Use of a fibrinogen receptor antagonist according to claims 1 or 2, characterised in that the compound (3S,5S)-5-[(4'-Amidino-4-biphenylyl)oxymethyl]-3-[(carboxy)methyl]-2-pyrrolidinone [3-$^3$H-4-biphenylyl] and the salts thereof are used as a labelled fibrinogen receptor antagonist.

10. Tritium-labelled fibrinogen receptor antagonist of general formula I according to claims 4 to 9, the tautomers thereof, the stereoisomers thereof including the mixtures and salts thereof.

11. Process for preparing the tritium-labelled fibrinogen receptor antagonist according to claim 10, characterised in that at least one tritium atom is introduced, instead of hydrogen, into a corresponding non-labelled fibrinogen receptor antagonist

        a) by catalytic tritiation of an unsaturated carboncarbon bond,

        b) by hydrogenolytic exchange of a halogen atom,

        c) by catalytic hydrogen/tritium exchange,

        d) by catalytic hydrogen/tritium exchange in tritiated solvents or

        e) by tritiating a precursor of the fibrinogen receptor antagonist which is to be produced and subsequently synthesising the fibrinogen receptor antagonist,

        and subsequently, if desired, an isomer mixture thus obtained is resolved into the enantiomers thereof and/or

        a tritiated compound thus obtained is converted into a salt thereof.

## Revendications

1. Utilisation d'un antagoniste du récepteur du fibrinogène qui présente pour le récepteur une affinité comparable ou supérieure au $^{125}$I-fibrinogène et qui, en présence de protéines étrangères, présente pour le récepteur une affinité ($K_D$) inférieure à 500 nM et qui contient au moins un atome de tritium détectable à la place de l'hydrogène, pour la détermination de la liaison de substances chimiques aux récepteurs du fibrinogène.

2. Utilisation d'un antagoniste du récepteur du fibrinogène qui présente pour le récepteur une affinité comparable ou supérieure au $^{125}$I-fibrinogène et qui, en présence de protéines étrangères, présente pour le récepteur une affinité ($K_D$) inférieure à 500 nM et qui contient au moins un atome de tritium détectable à la place de l'hydrogène, pour la détermination de la concentration d'antagonistes des récepteurs du fibrinogène.

3. Utilisation d'un antagoniste du récepteur du fibrinogène selon la revendication 1 ou 2, caractérisée en ce que la détermination est réalisée en présence de protéines étrangères et/ou de liquides de l'organisme.

4. Utilisation d'un antagoniste du récepteur du fibrinogène selon les revendications 1 ou 2, caractérisée en ce que l'on utilise comme antagoniste du récepteur du fibrinogène une amidine de formule générale

$$R_a - R_b \qquad\qquad (I)$$

dans laquelle

        $R_a$ représente un groupe 4-amidinophényle ou 5-amidinopyrimid-2-yle et
        $R_b$ représente un groupe HOOC-D-C-B-A dans lequel

        A représente un groupe phénylène éventuellement substitué par un groupe méthoxy, dans lequel, en outre, un ou deux groupes méthine peuvent être substitués chacun par un atome d'azote, un groupe imidazolinonediyle, imidazolidinonediyle, imidazolidinedionediyle, triazolinonediyle ou 1,1-dioxo-1,2,5-thiadiazolidinediyle éventuellement substitué sur un atome de carbone par un groupe méthyle, éthyle ou trifluorométhyle, un groupe benzimidazolediyle éventuellement substitué sur l'un des atomes d'azote par le reste $R_1$ ou un groupe aminocarbonyle lié au reste B par l'atome d'azote,
        B représente un groupe méthylène, carbonyle, cyclohexylène, phénylène ou imidazolediyle, un groupe aminocarbonyle lié au reste C par l'atome d'azote, qui peut en même temps être substitué sur l'atome d'azote par un groupe méthyle, ou un groupe méthylénoxy lié au reste A par l'atome d'oxygène,
        C représente un groupe éthylène éventuellement substitué par le reste $R_2$, un groupe cyclohexylène, un groupe pyrrolidinediyle ou pyrrolidinonediyle éventuellement substitué sur l'atome d'azote par le reste $R_3$, un groupe pipéridinediyle ou un groupe aminocarbonyle lié au reste D par l'atome d'azote et

D représente une liaison, un groupe méthylène ou éthylène,

$R_1$ représentant un groupe méthyle, 2-pipérazinoéthyle, 2-(3,4-diméthoxyphényl)éthyle ou 3-thiomorpholinopropyle,

$R_2$ un groupe amino ou hydroxyle et

$R_3$ un groupe 3-phénylpropyle, acétyle, méthanesulfonyle ou pyrrolidinocarbonylméthyle,

ses tautomères, ses stéréoisomères y compris ses mélanges et ses sels, dans laquelle au moins un atome d'hydrogène est remplacé par le tritium.

**5.** Utilisation d'un antagoniste du récepteur du fibrinogène selon les revendications 1 ou 2, caractérisée en ce que l'on utilise comme antagoniste du récepteur du fibrinogène une amidine de formule générale I selon la revendication 4, dans laquelle

$R_a$ représente un groupe 4-amidinophényle ou encore, lorsque

$R_b$ représente un groupe phényle substitué en position 4 par un groupe 3-carboxyméthylpyrrolidin-2-on-5-ylméthyloxy, un groupe 5-amidinopyrimid-2-yle et

$R_b$ représente un groupe phényle qui est substitué en position 4 par un groupe 4-carboxyméthylpyrrolidin-2-yl-méthyloxy, 3-carboxyméthylpyrrolidin-2-on-5-ylméthyloxy, 4-carboxyméthylpipéridinométhyle, 4-carboxyméthylpipéridinocarbonyle, 4-carboxycyclohexylaminocarbonyle ou N-méthyl-N-(4-carboxycyclohexyl)aminocarbonyle, dans chaque cas en position 1 la partie pyrrolidine pouvant être substituée par un groupe acétyle ou méthanesulfonyle et la partie pyrrolidinone pouvant être substituée par un groupe 3-phénylpropyle ou pyrrolidinocarbonylméthyle,

un groupe 4-méthoxyphényle qui est substitué en position 3 par un groupe 4-carboxycyclohexylaminocarbonyle,

un groupe pyridazin-3-yle substitué en position 6 par un groupe imidazol-1-yle, la partie imidazolyle étant substituée en position 4 par un groupe 2-amino-2-carboxyéthyle ou 2-carboxy-2-hydroxyéthyle,

un groupe imidazolidin-2-on-1-yle qui est substitué en position 3 par un groupe 4-(2-carboxyéthyl)cyclohexyle ou 4-(2-carboxyéthyl)phényle,

un groupe 4-méthyl-4-imidazolin-2-on-1-yle ou imidazolidine-2,4-dion-1-yle qui est substitué en position 3 par un groupe 4-(2-carboxyéthyl)phényle,

un groupe 1,2,4-triazol-5-in-3-on-2-yle, éventuellement substitué en position 5 par un groupe méthyle ou éthyle, qui est substitué en position 4 par un groupe 4-(2-carboxyéthyl)phényle,

un groupe 1,2,4-triazol-5-in-3-on-4-yle qui est substitué en position 2 par un groupe 4-(2-carboxyéthyl)phényle,

un groupe 1,1-dioxo-1,2,5-thiadiazolidin-2-yle substitué en position 5 par un groupe 4-(2-carboxyéthyl)phényle,

un groupe benzimidazol-2-yle éventuellement substitué en position 1 par un groupe méthyle, 2-pipérazinoéthyle ou 2-(3,4-diméthoxyphényl)éthyle, qui est substitué en position 5 par un groupe 2-carboxyéthylaminocarbonyle, ou

un groupe phénylaminocarbonyle qui est substitué en position 3 par un groupe 2-carboxyéthylaminocarbonyle,

ses tautomères, ses stéréoisomères y compris ses mélanges et ses sels, dans laquelle au moins un atome d'hydrogène est remplacé par le tritium.

**6.** Utilisation d'un antagoniste du récepteur du fibrinogène selon les revendications 1 ou 2, caractérisée en ce que l'on utilise comme antagoniste du récepteur du fibrinogène l'un des composés suivants de formule générale I selon la revendication 4 dans lesquels au moins un atome d'hydrogène est remplacé par le tritium:

(1) (3S,5S)- et (3R,5R)-5-[[(4-(5-amidinopyrimid-2-yl)phényl]oxyméthyl]-3-carboxyméthylpyrrolidin-2-one,

(2) (3S,5S)- et (3R,5R)-1-acétyl-5-[(4'-amidino-4-biphényl-yl)oxyméthyl]-3-carboxyméthylpyrrolidine,

(3) (3S,5S) - et (3R,5R)-5-[(4'-amidino-4-biphénylyl)oxyméthyl]-3-carboxyméthyl-1-méthanesulfonylpyrrolidine,

(4) (3S,5S) et (3R,5R)-5-[(4'-amidino-4-biphénylyl)oxyméthyl]-3-carboxyméthylpyrrolidin-2-one,

(5) 4-amidino-4'-[(trans-4-carboxycyclohexyl)aminocarbonyl]biphényle,

(6) 4-amidino-4'-[N-(trans-4-carboxycyclohexyl)-N-méthylaminocarbonyl]biphényle,

(7) 1-(4-amidinophényl)-3-[4-(2-carboxyéthyl)phényl]imidazolidine-2,4-dione,

(8) 1-(4-amidinophényl)-3-[4-(2-carboxyéthyl)phényl]-4-méthyl-4-imidazolin-2-one,

(9) 2-(4-amidinophényl)-4-[4-(2-carboxyéthyl)phényl]-1,2,4-triazol-5-in-3-one,

(10) 4-(4-amidinophényl)-2-[4-(2-carboxyéthyl)phényl]-1,2,4-triazol-5-in-3-one,

(11) 2-(4-amidinophényl)-4-[4-(2-carboxyéthyl)phényl]-5-méthyl-1,2,4-triazol-5-in-3-one,

(12) 2-(4-amidinophényl)-4-[4-(2-carboxyéthyl)phényl]-5-éthyl-1,2,4-triazol-5-in-3-one,

(13) 2-(4-amidinophényl)-5-[4-(2-carboxyéthyl)phényl]-1,2,5-thiadiazolidine-1,1-dioxyde,

(14) 1-(4-amidinophényl) -3-[4-(2-carboxyéthyl)cyclohexyl]imidazolidin-2-one,

(15) 2-(4-amidinophényl)-5-[(2-carboxyéthyl)aminocarbonyl]-1-[2-(pipérazin-1-yl)éthyl]benzimidazole,

(16) 4-amidino-4'-[4-(carboxyméthyl)pipéridinocarbonyl]biphényle et

leurs sels.

7. Utilisation d'un antagoniste du récepteur du fibrinogène selon les revendications 1 ou 2, caractérisée en ce que l'on utilise comme antagoniste du récepteur du fibrinogène l'un des composés suivants de formule générale I selon la revendication 4 dans lesquels au moins un atome d'hydrogène est remplacé par le tritium:

(1) (3S, 5S) - et (3R,5R)-5-[[(4-(5-amidinopyrimid-2-yl)phényl]oxyméthyl]-3-carboxyméthylpyrrolidin-2-one,

(2) (3S,5S) et (3R,5R)-1-acétyl-5-[(4'-amidino-4-biphényl-yl)oxyméthyl]-3-carboxyméthylpyrrolidine,

(3) (3S,5S) - et (3R,5R)-5-[(4'-amidino-4-biphénylyl)oxyméthyl]-3-carboxyméthyl-1-méthanesulfonylpyrrolidine,

(4) (3S,5S)- et (3R,5R)-5-[(4'-amidino-4-biphénylyl)oxyméthyl]-3-carboxyméthylpyrrolidin-2-one,

(5) 4-amidino-4'-[4-(carboxyméthyl)pipéridinocarbonyl]biphényle et

leurs sels.

8. Utilisation d'un antagoniste du récepteur du fibrinogène selon les revendications 1 ou 2, caractérisée en ce que, dans les antagonistes de récepteur de formule générale I selon les revendications 4 à 7, un atome d'hydrogène aromatique est replacé par le tritium.

9. Utilisation d'un antagoniste du récepteur du fibrinogène selon les revendications 1 ou 2, caractérisée en ce que l'on utilise comme antagoniste du récepteur du fibrinogène marqué le composé (3S,5S)-5-[(4'-amidino-4-biphénylyl)oxyméthyl]-3-[(carboxy)méthyl]-2-pyrrolidinone[3-$^3$H-4-biphénylyle] et ses sels.

10. Antagonistes du récepteur du fibrinogène marqués au tritium de formule générale I selon les revendications 4 à 9, leurs tautomères, leurs stéréoisomères y compris leurs mélanges et leurs sels.

11. Procédé de préparation des antagonistes du récepteur du fibrinogène marqués au tritium selon la revendication 10, caractérisé en ce que l'on introduit au moins un atome de tritium à la place de l'hydrogène dans un antagoniste du récepteur du fibrinogène non marqué correspondant

a) par tritiation catalytique d'une liaison carbone-carbone insaturée,

b) par échange hydrogénolytique d'un atome d'halogène,

c) par échange catalytique hydrogène/tritium,

d) par échange catalytique hydrogène/tritium dans des solvants tritiés ou

e) par tritiation d'un progéniteur de l'antagoniste du récepteur du fibrinogène qui doit être préparé puis synthèse de l'antagoniste du récepteur du fibrinogène, puis,

si on le souhaite, on résout un mélange d'isomères ainsi obtenu en ses énantiomères et/ou on convertit un composé tritié ainsi obtenu en son sel.

Abbildung I

**3H-BIBU 52 ZW-Bindung
Human-Thrombocyten (PRP)**

VE:504/06.09.91/Schubert

Abbildung 1. Verdrängung der Bindung von $^3$H-BIBU 52
an Humanthrombozyten in Gegenwart von
Plasma durch BIBU 52

| | |
|---|---|
| Gesamtaktivität gebunden: | 1576 cpm |
| Unspezifische Bindung: | 0 cpm |
| Dissoziationskonstante: | 58 nM |
| Bindungsstellen: | 67400 / Zelle |